# FASCICULE DE BREVET EUROPEEN

(11) **EP 2 667 796 B1**
(45) Date de publication et mention de la délivrance du brevet: **06.07.2016**
(21) Numéro de dépôt: 12702594.8
(22) Date de dépôt: 10.01.2012
(51) Int. Cl.: A61B 17/16, A61B 17/17, A61B 17/34, A61B 17/00

(54) **DISPOSITIF DE FORAGE POUR LA RÉALISATION D'UN CANAL OSSEUX À PROFIL COURBE À L'INTÉRIEUR DU CORPS D'UNE VERTÈBRE**
BOHRVORRICHTUNG ZUR HERSTELLUNG EINES KNOCHENKANALS MIT GEWÖLBTEM PROFIL IN EINEM WIRBELKÖRPER
DRILLING DEVICE FOR MAKING A BONE CANAL WITH A CURVED PROFILE INSIDE THE BODY OF A VERTEBRA

(30) Priorité: 24.01.2011 FR 1100199
(43) Date de publication de la demande: 04.12.2013
(73) Titulaire: Clariance, 62000 Dainville (FR)
(72) Inventeur: BILLON, Adrien, 59790 Rochin (FR); LEROY, Jean Yves, 62870 Campagne-les-hesdin (FR); TORNIER, Alain, 38330 Saint Ismier (FR); VIART, Guy, 62128 Saint Leger (FR); GANGI, Afshin, 77933 Lahr (DE); ORTIZ, Orlando, New York USA 11530 (US)
(74) Mandataire: Jeannet, Olivier
(86) Numéro de dépôt international: PCT/FR2012/050046
(87) Numéro de publication internationale: WO 2012/101354

(56) Documents cités:
- WO-A1-2004/043271
- US-A1- 2010 211 076

## Description

La présente invention est relative à un dispositif de forage pour la réalisation d'un canal osseux à profil courbe à l'intérieur du corps d'une vertèbre et notamment pour permettre l'accès au disque intervertébral et l'introduction d'un implant nucléique entre les corps vertébraux sus jacents et sous jacents d'un segment rachidien à instrumenter.

On connait d'après le document antérieur WO2004/043271 des dispositifs de perçage qui permettent au chirurgien d'accéder à l'espace intervertébral pour restaurer une configuration tridimensionnelle plus normal de l'espace, avec ou sans fusion en outre deux vertèbres adjacentes. Le dispositif de perçage est constitué d'un premier foret à os non flexible et d'un second foret souple introduit respectivement dans un tube de retenue qui est avancé à travers une canule transpédiculaire afin de réaliser l'espace entre les vertèbres.

On connait également d'après le document antérieur US2010/211076 des dispositifs de perçage qui permettent de créer une cavité dans les tissus osseux. Le dispositif de perçage repose sur un mécanisme d'entraînement permettant de former un profil qui permet la création d'un chemin ou d'une cavité dans l'os pour l'introduction de ciment osseux ou autre charge pour traiter une fracture ou une autre.

Le dispositif de forage suivant la présente invention a pour objet de perfectionner les moyens permettant la réalisation d'un canal osseux à profil courbe de manière que ledit canal débouche toujours suivant une direction sensiblement perpendiculaire à celle du plateau cortical de la vertèbre à percer.

La direction du canal osseux à profil courbe doit permettre l'introduction d'outils assurant une nucléotomie dans le disque intervertébral.

Le dispositif de forage suivant la présente invention comporte une broche de guidage issue d'un kit de broche de guidage et un foret articulé constitué d'un tube cylindrique comportant une extrémité agencée suivant un profil assurant la déformation et l'articulation suivant une forme courbe, ladite broche de guidage présentant à l'une de ses extrémités d'une part un profil courbe dont le rayon de courbure R est inférieur à 20 millimètres et d'autre part une pointe effilée se trouvant dans une direction définie par un angle Y qui est inférieur à 90 degrés par rapport à l'axe longitudinal de ladite broche, le profil de ladite broche de guidage permettant de définir, après son introduction dans le corps de la vertèbre, un premier point de **contact a** entre la broche et le foret articulé, ledit point de contact **a** étant positionné au niveau de la tangente au profil externe du rayon de courbure R de la broche de guidage dans une direction perpendiculaire au plateau cortical, et un second point de contact **b** entre la broche et le foret articulé, ledit point de contact **b** étant positionnée à l'extrémité libre dudit foret qui vient contre le profil externe de la pointe effilée de la broche de guidage se trouvant dans l'épaisseur du plateau cortical, lesdits deux points de contact **a** et **b** assurant le guidage de l'extrémité libre du foret articulé afin de positionner ladite extrémité libre dans une direction sensiblement perpendiculaire à celle du plateau cortical de la vertèbre à percer.

Le dispositif de forage suivant la présente invention comporte une broche de guidage qui est réalisée dans un matériau à la fois résistant et flexible tel que dans un alliage en Nitinol super élastique.

Le dispositif de forage suivant la présente invention comporte une broche de guidage dont le diamètre externe est inférieur à 3 millimètres.

Le dispositif de forage suivant la présente invention comporte une broche de guidage dont le diamètre externe est compris entre 1.4 millimètres et 2 millimètres.

Le dispositif de forage suivant la présente invention comporte une broche de guidage dont le profil courbe présente un rayon de courbure R qui est compris entre 10 millimètres et 20 millimètres.

Le dispositif de forage suivant la présente invention comporte un angle Y de la pointe effilée qui est compris entre 70 et 85 degrés.

Le dispositif de forage suivant la présente invention comporte une broche de guidage comprenant une partie longitudinale droite présentant un méplat coopérant avec un profil complémentaire ménagé dans un porte broche permettant d'assurer d'une part le blocage en rotation de la broche de guidage à l'intérieur du porte broche et d'autre part de rigidifier ladite broche de guidage sur sa partie longitudinale droite lors de l'introduction de ladite broche de guidage et dudit porte broche au travers d'une canule droite préalablement ancrée dans le corps de la vertèbre.

Le dispositif de forage suivant la présente invention comporte une broche de guidage comprenant au niveau de son extrémité opposée à celle à profil courbe une encoche ménagée sur le pourtour de la partie longitudinale droite et plus particulièrement du coté du méplat, ladite encoche coopérant avec une mollette de serrage guidée dans une poignée de préhension du porte broche pour le blocage en translation de ladite broche de guidage dans ledit porte broche.

Le dispositif de forage suivant la présente invention comporte un porte broche qui est constitué d'un tube cylindrique et métallique solidaire à l'une de ses extrémités d'une poignée de préhension pourvue d'une molette de serrage et d'un manchon cylindrique creux présentant sur son profil externe un filetage rapide permettant le vissage d'un bouchon permettant de bloquer en translation la broche de guidage par rapport au porte broche.

Le dispositif de forage suivant la présente invention comporte un foret articulé qui est constitué d'un tube cylindrique et métallique comportant à l'une de ses extrémités une poignée de préhension, tandis que l'autre extrémité est découpée d'une part suivant un profil assurant la déformation et l'articulation suivant une forme courbe de ladite extrémité et d'autre part pour déterminer une extrémité coupante comprenant une première série de dents disposée sur la périphérie dudit tube et une seconde série de dents disposée en bout du tube métallique et d'une gaine de protection placée dans la partie interne du tube métallique, ladite gaine de protection étant réalisée en matériau souple permettant de lisser intérieurement les aspérités et les discontinuités de l'extrémité libre provenant du profil.

Le dispositif de forage suivant la présente invention comporte un foret articulé dont la poignée de préhension comprend dans le prolongement du tube métallique un manchon cylindrique creux présentant sur son profil externe un filetage rapide permettant l'introduction et la fixation d'une gaine de centrage à l'intérieur dudit tube métallique.

Le dispositif de forage suivant la présente invention comporte une gaine de protection qui est réalisée dans un matériau souple assurant un coefficient de glissement important pour le coulissement de la broche de guidage et/ou de la gaine de centrage lors du forage du canal osseux à profil courbe.

Le dispositif de forage suivant la présente invention comporte un foret articulé dont le profil du tube cylindrique et métallique est constitué d'une succession de boucles alternativement concaves et convexes assurant la déformation et l'articulation suivant une forme courbe de l'extrémité du foret articulé.

Le dispositif de forage suivant la présente invention comporte un foret articulé dont le bord tranchant de chaque dent de la première série de dents présente une faible inclinaison par rapport à l'axe longitudinal du foret articulé.

Le dispositif de forage suivant la présente invention comporte un foret articulé dont le bord tranchant de chaque dent de la seconde série de dents présente une forte inclinaison de manière à couper l'axe longitudinal du foret articulé.

Le dispositif de forage suivant la présente invention comporte une gaine de centrage qui est constituée d'un tube cylindrique réalisé dans un matériau souple solidaire à l'une de ses extrémités d'une tête de préhension comportant d'une part un alésage interne fileté destiné à coopérer avec le manchon de la poignée du foret articulé pour son immobilisation sur ce dernier et d'autre part à l'opposé de l'alésage interne fileté un dispositif de blocage permettant par pincement, de bloquer en translation le tube cylindrique à l'intérieur de ladite tête de la gaine de centrage.

Le dispositif de forage suivant la présente invention comporte un foret articulé comprenant un dispositif de verrouillage disposé sur le tube métallique et en dessous de la poignée de préhension afin de pouvoir, au moyen d'un élément de retenue et d'un écrou de serrage, immobiliser ledit foret sur la canule droite préalablement ancrée dans le corps de la vertèbre.

Le dispositif de forage suivant la présente invention comporte un dispositif de verrouillage qui est constitué d'un élément de retenue comportant une tête cylindrique solidaire d'un manchon présentant un profil externe cylindrique pourvu d'un filetage se prolongeant par un profil externe conique fendu.

Le dispositif de forage suivant la présente invention comporte un dispositif de verrouillage dont l'élément de retenue est percé en son milieu d'un alésage débouchant comportant au niveau de la tête cylindrique un filetage interne coopérant avec un profil fileté de la canule droite pour l'immobilisation de l'élément de retenue sur ladite canule.

Le dispositif de forage suivant la présente invention comporte un dispositif de verrouillage dont l'écrou de serrage comprend dans sa partie interne un premier alésage fileté coopérant avec celui ménagé sur la partie cylindrique du manchon de l'élément de retenue et un second alésage interne coaxial au premier et présentant un profil conique d'inclinaison complémentaire à celui externe dudit manchon.

Le dispositif de forage suivant la présente invention comporte un foret articulé comprenant des moyens de sécurité assurant la récupération de l'extrémité coupante en cas de rupture dudit foret articulé.

Le dispositif de forage suivant la présente invention comporte un foret articulé dont les moyens de sécurité sont constitués d'un fil souple ou câble souple placé à l'intérieur du tube métallique et plus particulièrement entre la gaine de protection solidaire de la face interne dudit tube et la face externe de la gaine de centrage de manière que chaque extrémité dudit fil souple coopère avec la tête de préhension de ladite gaine de centrage.

Le dispositif de forage suivant la présente invention comporte un foret articulé dont les moyens de sécurité sont constitués d'un fil souple ou câble souple placé à l'intérieur du tube métallique et entre la face interne de ce dernier et la gaine de protection de manière que chaque extrémité dudit fil souple coopère avec la tête de préhension de ladite gaine de centrage.

Le dispositif de forage suivant la présente invention comporte un fil souple de sécurité dont les extrémités comprennent respectivement une butée placée dans un logement de même profil ménagé dans la tête de préhension de la gaine de centrage.

La description qui va suivre en regard des dessins annexés, donnés à titre d'exemples non limitatifs, permettra de mieux comprendre l'invention, les caractéristiques qu'elle présente et les avantages qu'elle est susceptible de procurer :
Figures 1 et 1 a sont des vues en perspective illustrant le positionnement de la canule fixée dans le corps vertébral d'un segment rachidien pour l'introduction des éléments constituant le dispositif de forage suivant la présente invention.
Figures 2, 2a, 2b sont des vues représentant la broche de guidage du dispositif de forage suivant la présente invention.
Figures 3, 3a, 3b sont des vues illustrant le préhenseur de broche pour la mise en place de la broche de guidage du dispositif de forage suivant la présente invention.
Figures 4, 4a à 4d sont des vues montrant un forêt articulé du dispositif de forage suivant la présente invention permettant le perçage d'un canal courbe dans le corps vertébral d'un segment rachidien.
Figures 4e à 4j sont des vues illustrant une variante du foret articulé du dispositif de forage suivant la présente invention, ledit foret comportant des moyens de sécurité assurant la récupération de l'extrémité coupante en cas de rupture du foret articulé.
Figures 5 et 5c sont des vues représentant une gaine de centrage pour la mise en place du foret articulé autour de la broche de guidage et dans la canule du dispositif de forage suivant la présente invention.
Figures 5a, 5b, 5d et 5e sont des vues montrant un dispositif de verrouillage permettant d'immobiliser le foret articulé sur la canule droite du dispositif de forage suivant la présente invention.
Figures 6 à 13 sont des vues illustrant les différentes étapes permettant la mise en place de la broche de guidage à l'intérieur du corps vertébral d'une vertèbre au moyen du dispositif de forage suivant la présente invention.
Figures 14 à 20 sont des vues représentant les différentes étapes permettant la réalisation d'un canal à profil courbe dans le corps vertébral d'une vertèbre pour atteindre la face supérieure du disque intervertébral au moyen d'un foret articulé du dispositif de forage suivant la présente invention.
Figure 21 est une vue montrant les zones de contact du foret articulé par rapport à la broche de guidage du dispositif de forage suivant la présente invention permettant de positionner l'extrémité libre dudit foret articulé dans une direction sensiblement perpendiculaire au plateau de la vertèbre à percer.

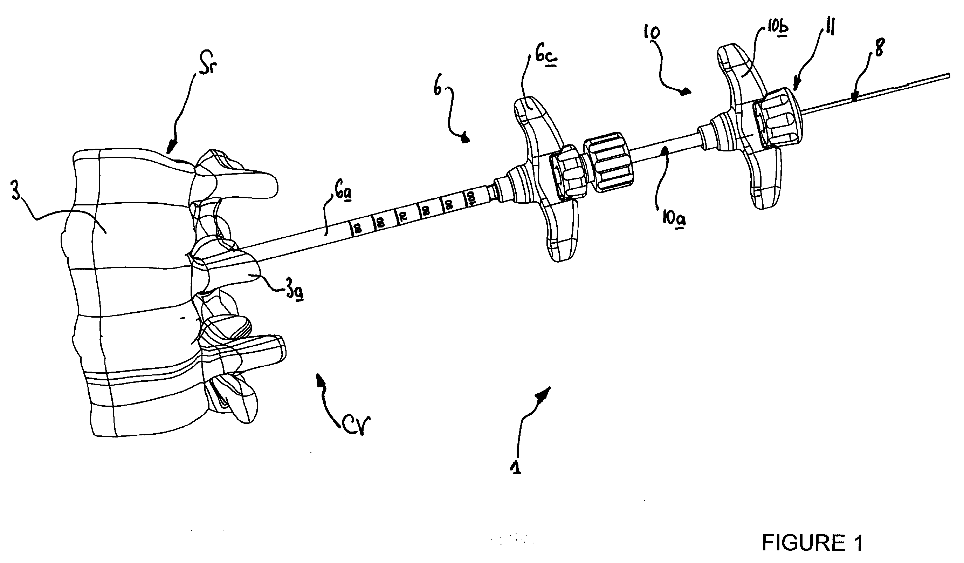

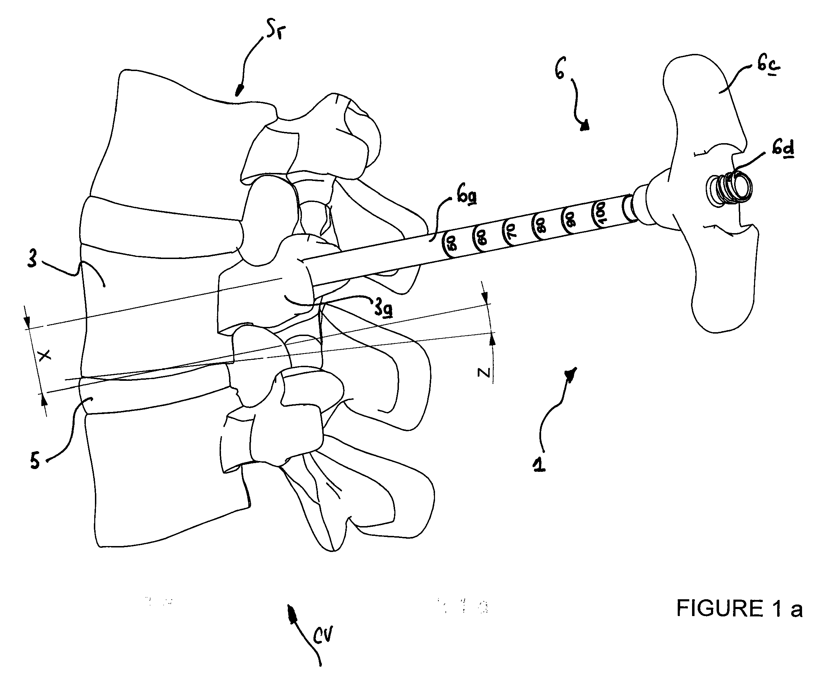

On a montré en figures 1, 1 a et 20 un dispositif de forage 1 comportant un ensemble d'élément agencés entre eux permettant la réalisation d'un canal osseux à profil courbe 2 à l'intérieur du corps d'une vertèbre 3 et du plateau cortical 4 d'un segment rachidien Sr d'une colonne vertébrale Cv afin d'atteindre la face supérieure d'un disque intervertébral 5 endommagé.

Le dispositif de forage 1 suivant la présente invention permet de réaliser un abord chirurgical percutané trans-osseux pour atteindre le nucleus du disque intervertébral 5 de manière à pouvoir par la suite effectuer les interventions nécessaires sur le disque intervertébral endommagé.

La canule droite 6 est vissée dans le haut du pédicule 3a de la vertèbre 3 correspondante pour avoir la plus grande distance x et le plus grand angle z entre ladite canule et le plateau inférieur de ladite vertèbre (Figure 1 a).

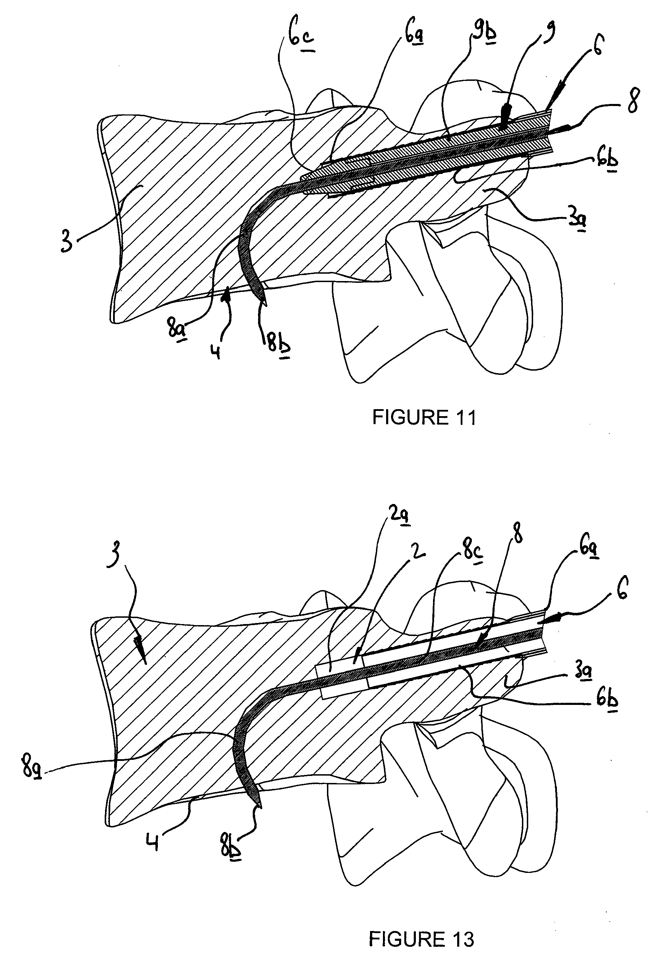

La canule droite 6 est constituée d'un tube cylindrique 6a comportant sur son profil externe au niveau de son extrémité libre un filetage 6b pour sa retenue dans le canal droit 2a préalablement réalisé (Figure 13).

Le tube cylindrique 6a est solidaire, à l'extrémité opposée à celle portant le filetage 6b, d'une poignée de préhension 6c en matière plastique de couleur. Le tube cylindrique 6a se prolonge au delà de la poignée de préhension 6b en présentant un profil fileté 6d permettant la fixation des autres éléments constituant le dispositif de forage 1 suivant la présente invention.

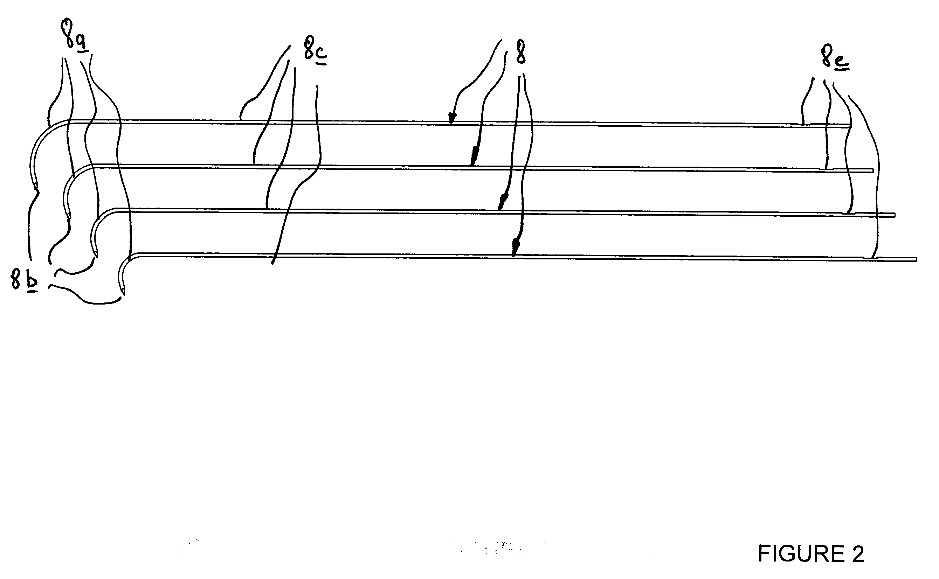

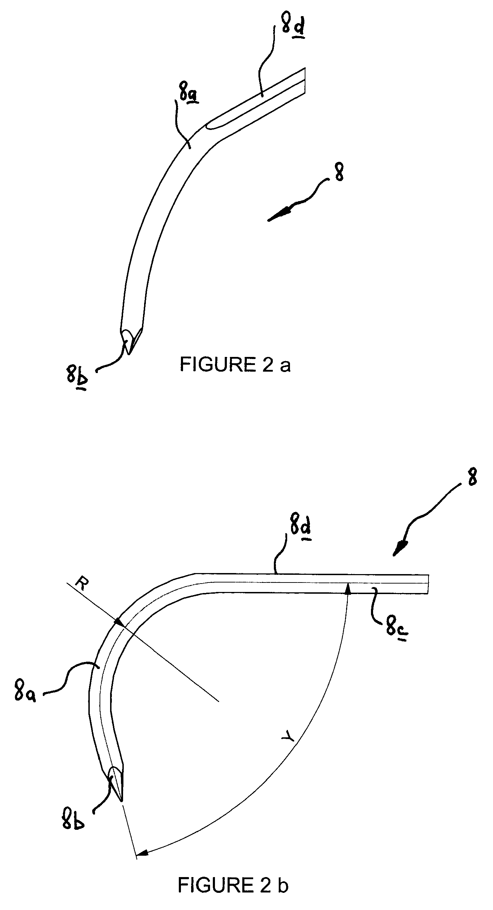

On a représenté en figures 2, 2a, 2b un kit de broches de guidage 8 permettant au moyen de la canule droite 6 préalablement fixée dans le corps de la vertèbre 3 d'atteindre le centre du disque intervertébral 5.

Chaque broche de guidage 8 est réalisée dans un matériau à la fois résistant et flexible tel que par exemple un alliage en Nitinol super élastique.

Chaque broche de guidage 8 présente un diamètre externe qui est inférieur à 3 millimètres_et à l'une de ses extrémités un profil courbe 8a dont le rayon de courbure R est inférieur à 20 millimètres.

Selon un mode de réalisation préféré, la broche de guidage 8 présente un diamètre externe qui est compris entre 1.4 millimètres et 2 millimètres et à l'une de ses extrémités un profil courbe 8a dont le rayon de courbure R est compris entre 10 millimètres et 20 millimètres.

Chaque broche de guidage 8 comporte au niveau de son extrémité à profil courbe 8a une pointe effilée 8b se trouvant dans une direction qui est définie par un angle Y qui est inférieur à 90 degrés par rapport à l'axe longitudinal de ladite broche.

Selon un mode de réalisation préféré chaque broche de guidage 8 comporte au niveau de son extrémité à profil courbe 8a une pointe effilée 8b se trouvant dans une direction qui est définie par un angle Y qui est compris entre 70 et 85 degrés par rapport à l'axe longitudinal de ladite broche.

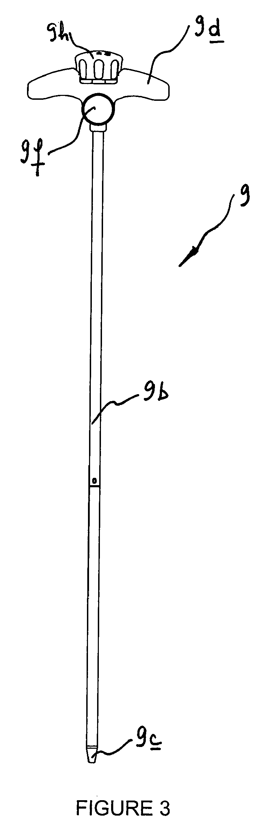

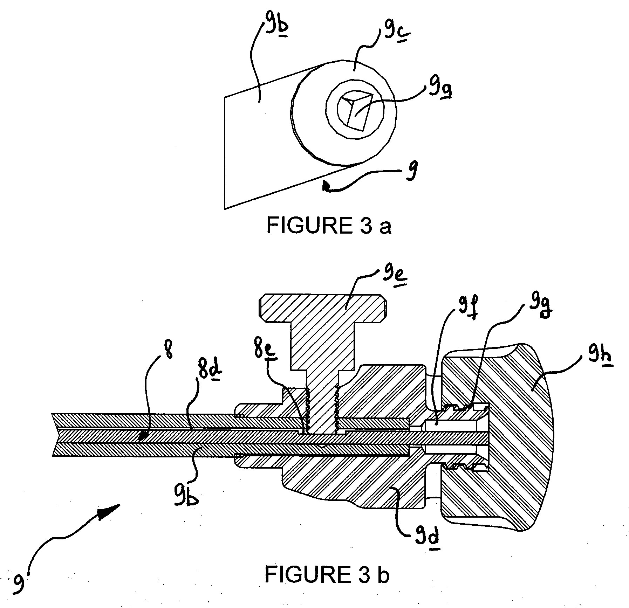

Chaque broche de guidage 8 comporte une partie longitudinale droite 8c présentant un méplat 8d coopérant avec un profil complémentaire 9a ménagé dans un porte broche 9 (Figures 3, 3a) permettant d'assurer d'une part le blocage en rotation de la broche de guidage 8 à l'intérieur du porte broche 9 et d'autre part de rigidifier ladite broche de guidage 8 sur sa partie longitudinale droite 8c lors de son introduction dans le corps vertébral 3 au travers de la canule droite 6 préalablement ancrée.

Chaque broche de guidage 8 comporte, au niveau de son extrémité opposée à celle à profil courbe 8a, une encoche 8e ménagée dans la partie longitudinale droite 8c et plus particulièrement du coté du méplat 8d.

Une broche de guidage 8 à profil courbe 8a avec un rayon de courbure R adapté est choisie en fonction de la distance X entre la canule droite 6 et le plateau inférieur de la vertèbre 3 correspondante.

On a représenté en figures 3, 3a et 3b le porte broche 9 qui est constitué d'un tube cylindrique et métallique 9b présentant une extrémité libre pourvue d'une part extérieurement d'un cône 9c permettant son glissement à l'intérieur de la canule 6 et d'autre part intérieurement le méplat 9a de profil complémentaire à celui de la broche de guidage 8.

La combinaison des méplats entre le porte broche 9 et la broche de guidage 8 permet d'orienter ladite broche de guidage 8 dans la canule droite 6 et de déterminer une position de sortie constante et toujours identique de ladite broche de guidage.

Le tube cylindrique 9b du porte broche 9 est solidaire, à l'opposé de son extrémité libre, d'une poignée de préhension 9d en matière plastique de couleur pourvue d'une molette de serrage 9e.

La molette de serrage 9e est prévue pour traverser le tube cylindrique 9b et venir déboucher à l'intérieur de l'encoche 8e ménagée dans la broche de guidage 8 afin de bloquer cette dernière en translation et en rotation à l'intérieur dudit porte broche 9.

La poignée de préhension 9d comporte dans le prolongement du tube cylindrique 9b un manchon cylindrique creux 9f traversé par la broche de guidage 8. Le manchon cylindrique 9f présente sur son profil externe un filetage rapide 9g permettant le vissage d'un bouchon 9h en matière plastique de couleur.

Le bouchon 9h après son vissage sur la poignée de préhension 9d vient en appui contre la broche de guidage permettant de bloquer temporairement en translation ladite broche de guidage 8 par rapport au porte broche 9 (Figure 3b).

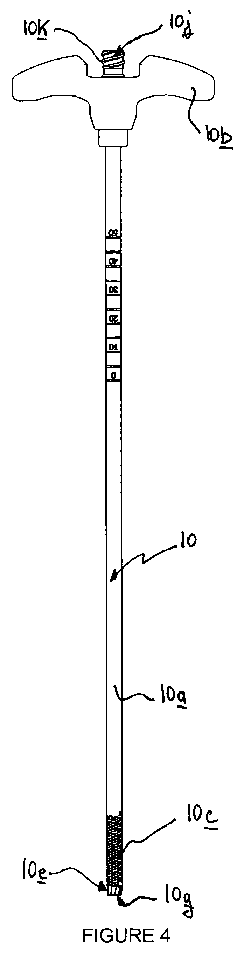

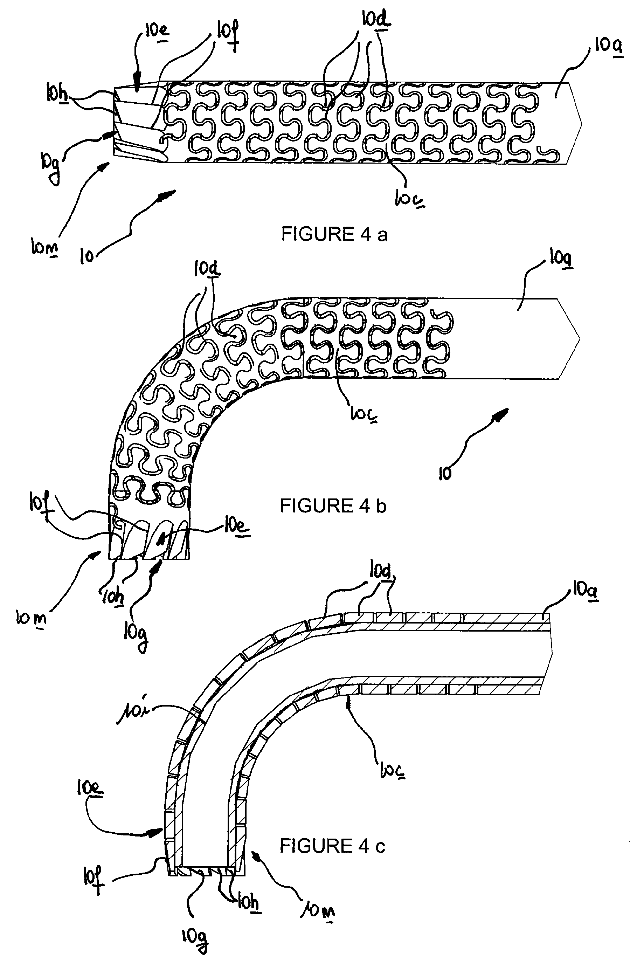

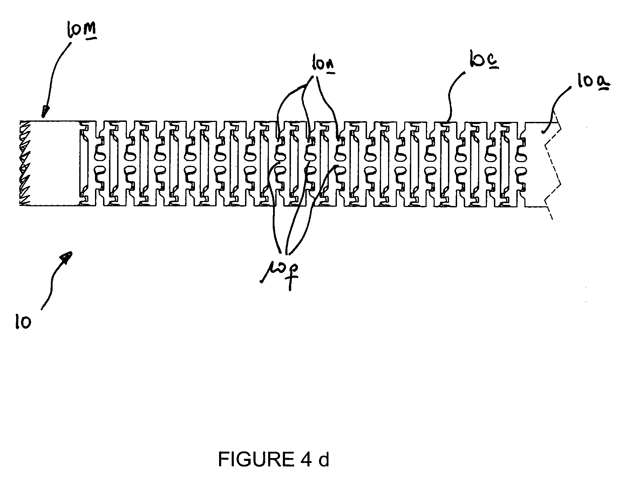

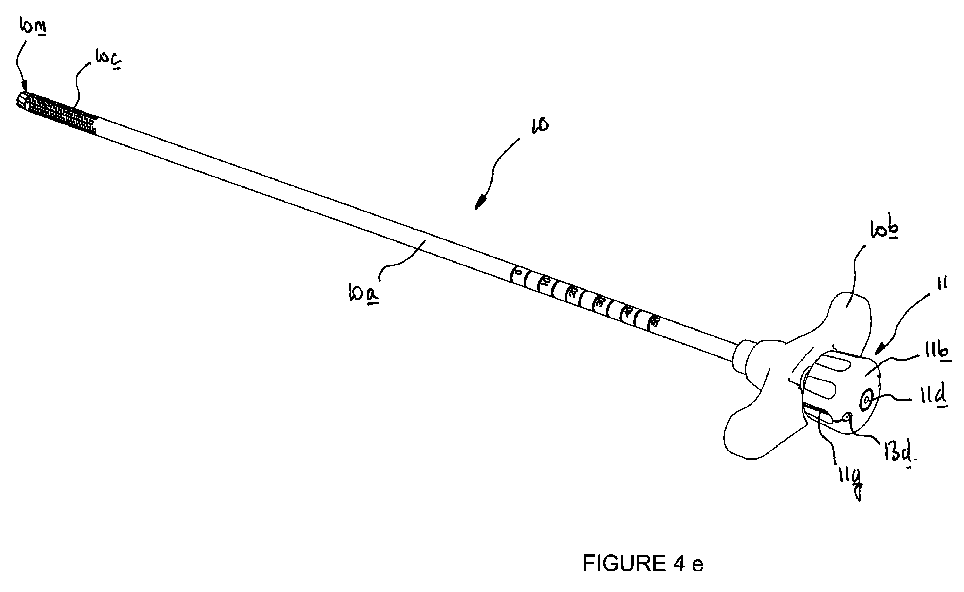

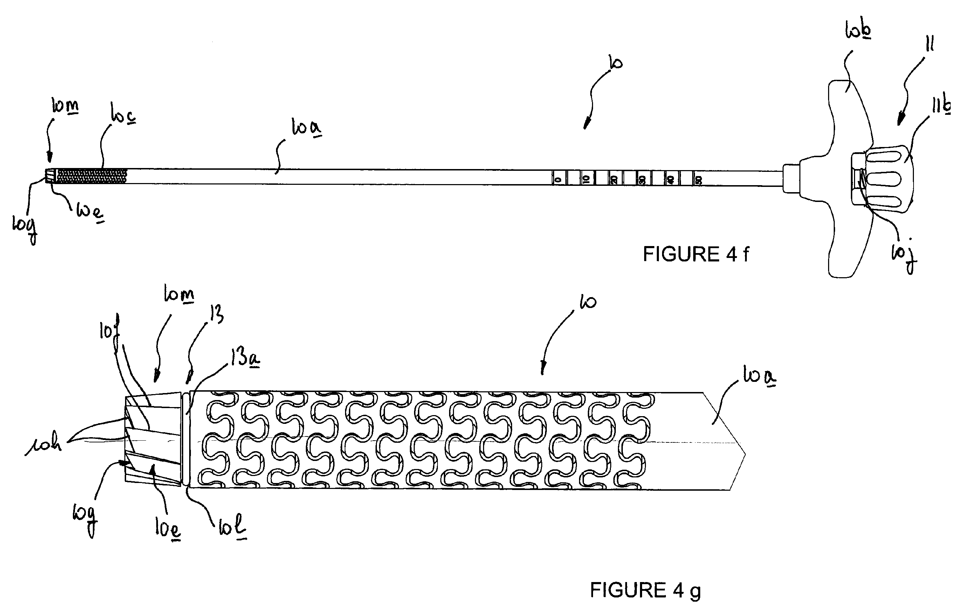

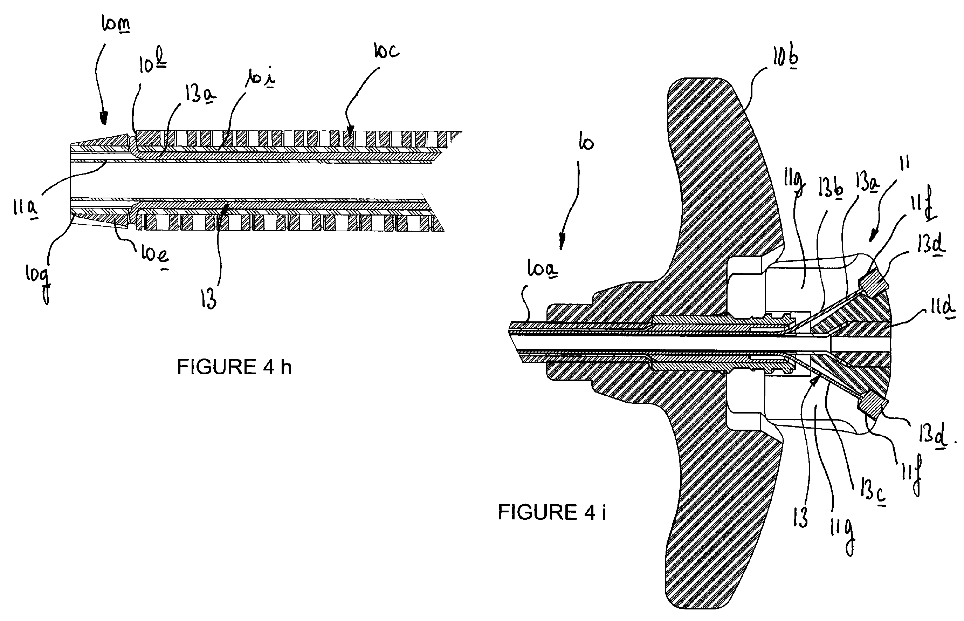

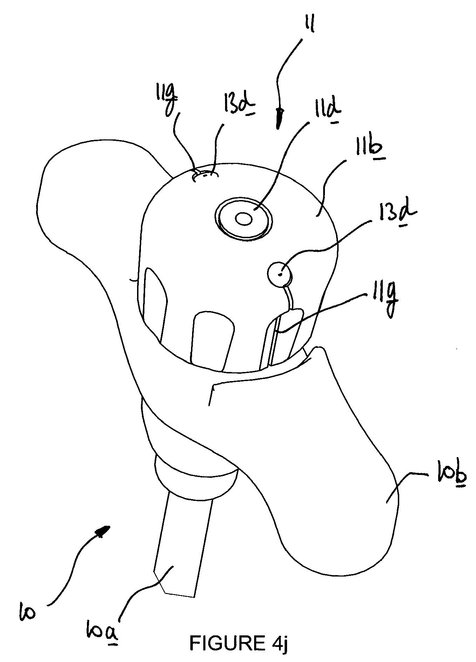

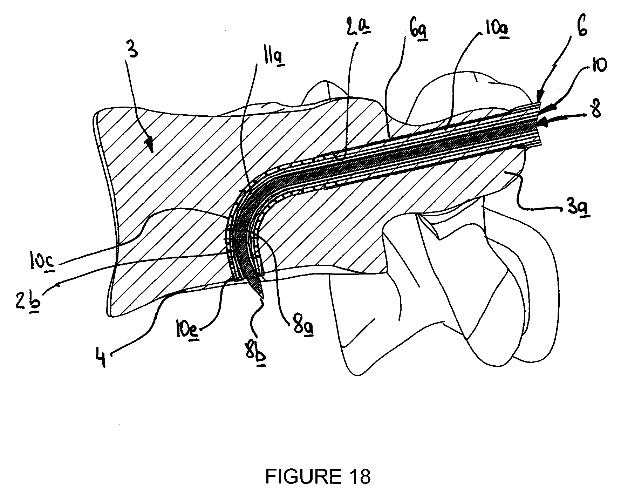

On a montré en figures 4, 4a à 4d et 18 un foret articulé 10 du dispositif de forage 1 suivant la présente invention qui est destiné à prolonger le canal osseux droit 2a préalablement réalisé par un canal à profil courbe 2b débouchant au dessus du disque intervertébral 5.

Le foret articulé 10 est constitué d'un tube cylindrique et métallique 10a comportant à l'une de ses extrémités une poignée de préhension 10b en matière plastique de couleur, tandis que l'autre extrémité est découpée suivant un profil 10c qui peut être constitué par exemple d'une succession de boucles 10d alternativement concaves et convexes assurant la déformation et l'articulation suivant une forme courbe de ladite extrémité.

Le tube métallique 10a se termine après le profil 10c par une extrémité coupante 10m comportant une première série de dents 10e disposée sur la périphérie dudit tube et dont le bord tranchant 10f de chaque dent présente une faible inclinaison par rapport à l'axe longitudinal du foret articulé 10.

L'extrémité coupante 10m comporte une seconde série de dents 10g qui est disposée en bout du tube métallique10a et dont chaque bord tranchant 10h présente une forte inclinaison de manière à couper l'axe longitudinal du foret articulé 10.

Le foret articulé 10 comporte dans la partie interne du tube métallique 10a une gaine de protection 10i en matériau souple permettant de lisser intérieurement les aspérités et les discontinuités de l'extrémité libre provenant du profil 10c formé par exemple par la succession de boucles 10d alternativement concaves et convexes.

La gaine de protection 10i peut être réalisée, par exemple, dans un matériau souple tel que du PTFE assurant également un coefficient de glissement important permettant le coulissement soit de la broche de guidage 8 soit d'une gaine de centrage 11 lors du forage du canal courbe 2b (Figure 4c).

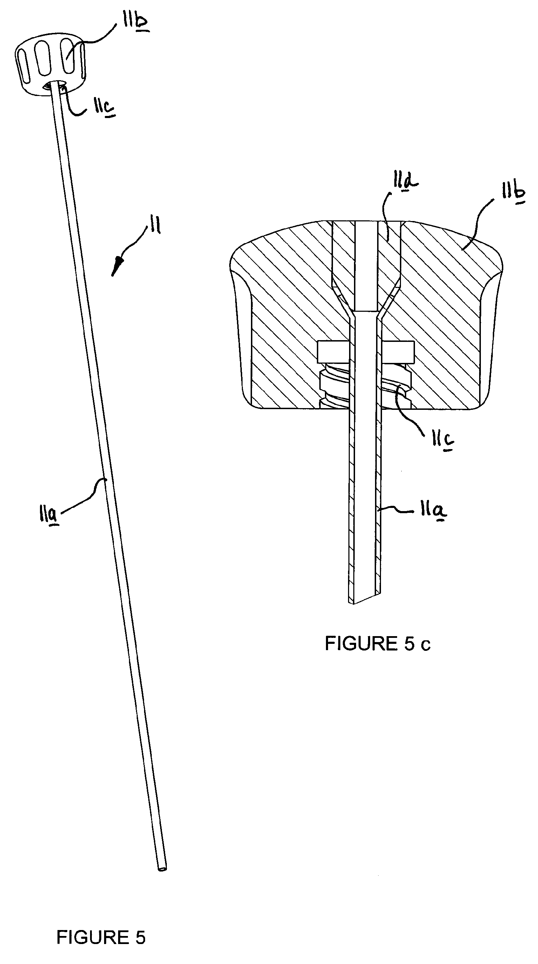

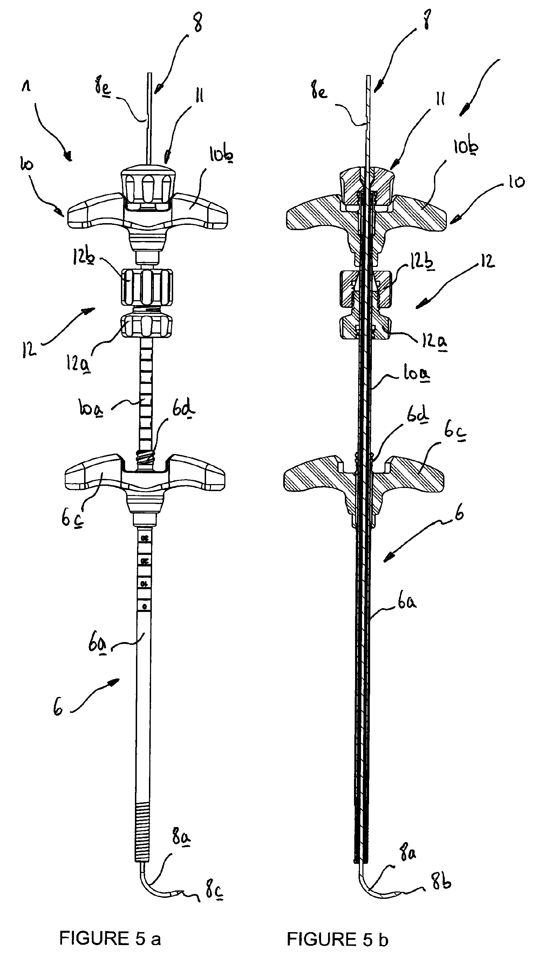

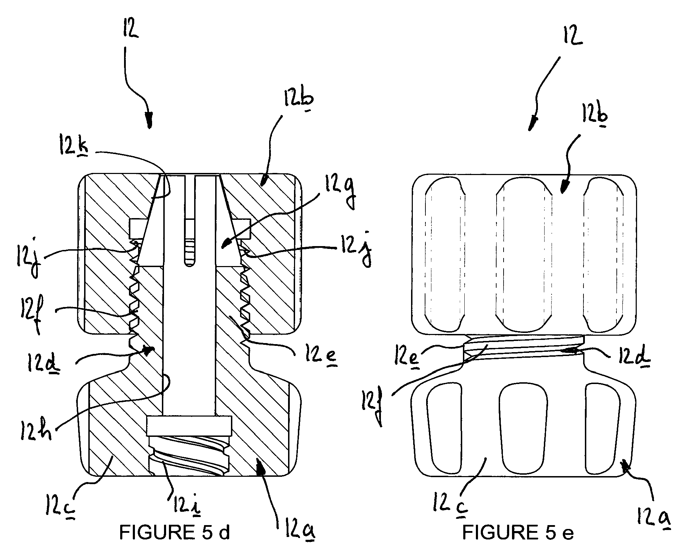

La poignée de préhension 10b comporte dans le prolongement du tube cylindrique 10a un manchon cylindrique creux 10j présentant sur son profil externe un filetage rapide 10k permettant le vissage d'une gaine de centrage 11 (Figures 4 et 5).

On a représenté en Figure 4d une variante de réalisation du profil 10c prévu à l'extrémité libre du tube métallique 10a du foret articulé 10. Le profil 10c est obtenu à partir d'une découpe de type cardan comportant des butées 10n entre chaque pont de liaison 10p. Les butées 10n permettent de réaliser un blocage empêchant le profil 10c de se déformer de manière trop importante lors de sa déformation suivant une forme courbe.

On a illustré en figures 4e à 4j une variante du foret articulé 10 qui peut comporter des moyens de sécurité 13 assurant la récupération de l'extrémité coupante 10m en cas de rupture dudit foret articulé.

Les moyens de sécurité 13 sont constitués d'un fil souple ou câble souple 13a placé à l'intérieur du tube métallique 10a et selon un premier mode de réalisation ledit fil souple ou câble souple 13a peut être placé entre la gaine de protection 10i solidaire de la face interne dudit tube et la face externe de la gaine de centrage 11.

Selon un second mode de réalisation le fil souple ou câble souple 13a peut être disposé entre la face interne du tube métallique et la gaine de protection 10i.

Le fil souple de sécurité 13a débouche à l'extérieur du tube métallique 10a d'une part au niveau du manchon cylindrique 10j de manière que chaque extrémité 13b, 13c dudit fil souple 13a coopère avec la tête de préhension 11b de la gaine de centrage 11 et d'autre part dans une gorge périphérique 10l ménagée entre l'extrémité coupante 10m et le profil 10c assurant la déformation dudit foret 10.

Le fil souple de sécurité 13a est passé dans deux petit trous ménagés dans la gaine de protection 10i et le tube métallique 10a pour déboucher à l'intérieur de la gorge périphérique 10l afin que ledit fil souple forme une boucle de sécurité entre l'extrémité coupante 10m et le profil 10c assurant la déformation dudit foret 10 tandis que le fil souple 13a ressort en deux brins dont chacune des extrémités 13b, 13c est logée dans la tête de préhension 11b de la gaine de centrage 11.

Les extrémités 13b, 13c du fil souple de sécurité 13a comportent respectivement une butée 13d placée dans un logement 11f de même profil ménagé dans la tête de préhension 11b. Cette dernière comporte des fentes 11g diamétralement opposées et débouchant dans chaque logement 11f pour permettre la mise en place des deux brins du fil souple de sécurité 13a.

Le fil souple de sécurité 13a relie l'extrémité coupante 10m du foret articulé 10 à l'extérieur de ce dernier pour permettre, en cas de rupture dudit foret, l'extraction complète de ce dit foret sans laisser de débris à l'intérieur du corps vertébral 3.

Egalement, lorsque le perçage du canal courbe 2b est réalisé sans rupture du foret articulé 10 le fil souple de sécurité 13a est retiré dudit foret après l'élimination de l'une au moins des butées 13d et par simple traction sur l'un des brins dudit fil souple.

On a montré en Figure 5 la gaine de centrage 11 qui est constituée d'un tube cylindrique 11a réalisé dans un matériau souple tel que du PTFE solidaire à l'une de ses extrémités d'une tête de préhension 11 b en matière plastique de couleur.

La tête de préhension 11b comporte du coté du tube cylindrique 11a un alésage interne fileté 11c destiné à coopérer avec le manchon 10j de la poignée 10b du foret articulé 10 pour son immobilisation sur ce dernier (figure 5c).

Egalement, la tête de préhension 11b comporte à l'opposé de l'alésage interne fileté 11c un dispositif de blocage 11d permettant par pincement, de bloquer en translation le tube cylindrique 11a à l'intérieur de ladite tête de la gaine de centrage 11 (figure 5c).

La gaine de centrage 11 permet d'une part de rattraper le jeu fonctionnel entre la broche de guidage 8 et le foret articulé 10 et d'autre part d'éloigner les dents 10g à bord tranchant 10h du foret articulé 10 de la broche de guidage 8 lors du forage du canal courbe 2b.

On a montré en figures 5a, 5b, 5d et 5e un dispositif de verrouillage 12 disposé sur le tube métallique 10a et en dessous de la poignée de préhension 10b du foret articulé 10 du dispositif de forage 1.

Le dispositif de verrouillage 12 peut librement coulisser sur le tube métallique 10a pour venir se visser sur le profil fileté 6d de la canule droite 6 lorsque le foret articulé 10 est bien positionné afin d'éviter tout déplacement de ce dernier après le perçage du canal courbe 2b.

Le dispositif de verrouillage 12 est constitué d'un élément de retenue 12a et d'un écrou de serrage 12b permettant de bloquer l'ensemble sur le tube métallique 10a du foret articulé 10. L'élément de retenue 12a comporte une tête cylindrique 12c solidaire d'un manchon 12d présentant un profil externe cylindrique 12e pourvu d'un filetage 12f se prolongeant par un profil externe conique fendu 12g.

L'élément de retenue 12a est percé en son milieu d'un alésage débouchant 12h comportant au niveau de la tête cylindrique 12c un filetage interne 12i coopérant avec le profil fileté 6d de la canule droite 6 pour l'immobilisation dudit élément de retenue sur ladite canule.

L'écrou de serrage 12b comporte dans sa partie interne un premier alésage fileté 12j coopérant avec celui 12f ménagé sur la partie cylindrique 12e du manchon 12d de l'élément de retenue 12a et un second alésage interne 12k coaxial au premier et présentant un profil conique d'inclinaison complémentaire à celui externe 12g dudit manchon 12d.

Le dispositif de verrouillage 12 est préalablement assemblé autour du tube métallique 10a du foret articulé 10 c'est-à-dire que l'écrou de serrage 12b est vissé sur l'élément de retenue 12a sans exercer de pression de blocage sur l'alésage interne débouchant 12h qui est en contact avec le pourtour externe dudit tube métallique 10a.

En effet, la pression de blocage provient du serrage complet de l'écrou de serrage 12b sur l'élément de retenue 12a de manière que l'alésage conique 12k vienne en appui sur le profil conique externe 12g du manchon 12d permettant d'engendrer une déformation de l'alésage interne 12h et donc une pression de blocage sur le pourtour externe du tube métallique 10a.

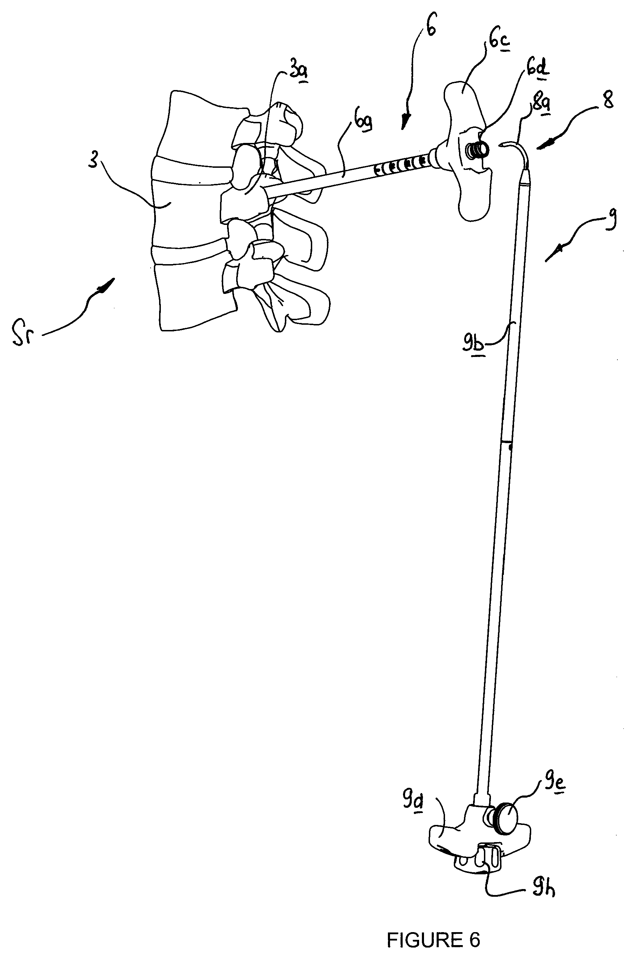

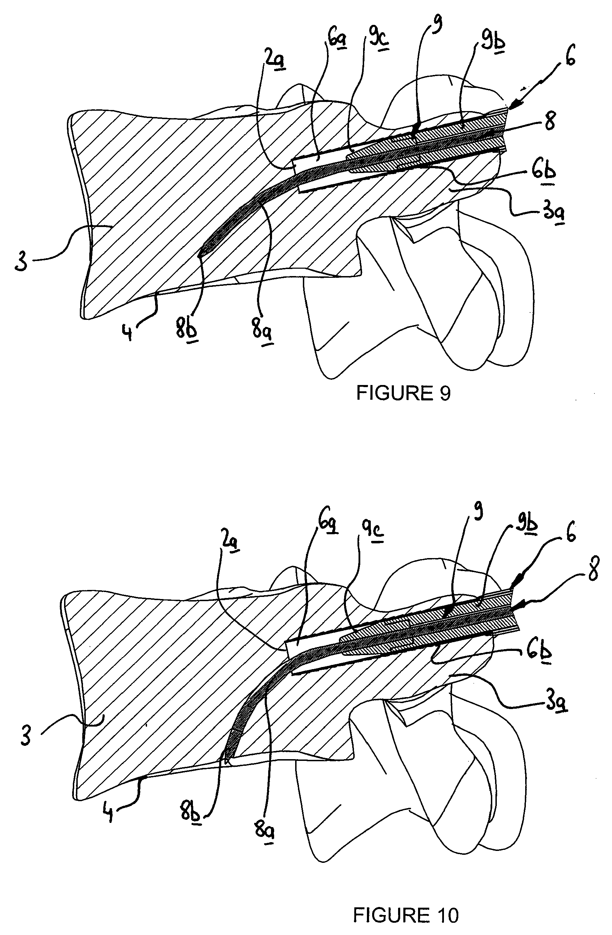

On a représenté en figures 6 à 13 les différentes étapes permettant la mise en place d'une broche de guidage 8 provenant du kit dont les caractéristiques relatives au rayon de courbure R et à l'angle Y de la pointe effilée 8b ont été préalablement déterminées en fonction des dimensions de la vertèbre 3 à percer et de la position du disque intervertébral 5.

Pour cela, la canule droite 6 est préalablement ancrée dans le canal 2a ménagé dans le haut du pédicule 3a et en direction du centre de la vertèbre 3 du segment rachidien Sr.

La broche de guidage 8 correspondante est introduite dans le porte broche 9 et immobilisée en translation et rotation par rapport à ce dernier par l'intermédiaire de la molette de serrage 9e et du bouchon 9h (Figure 6).

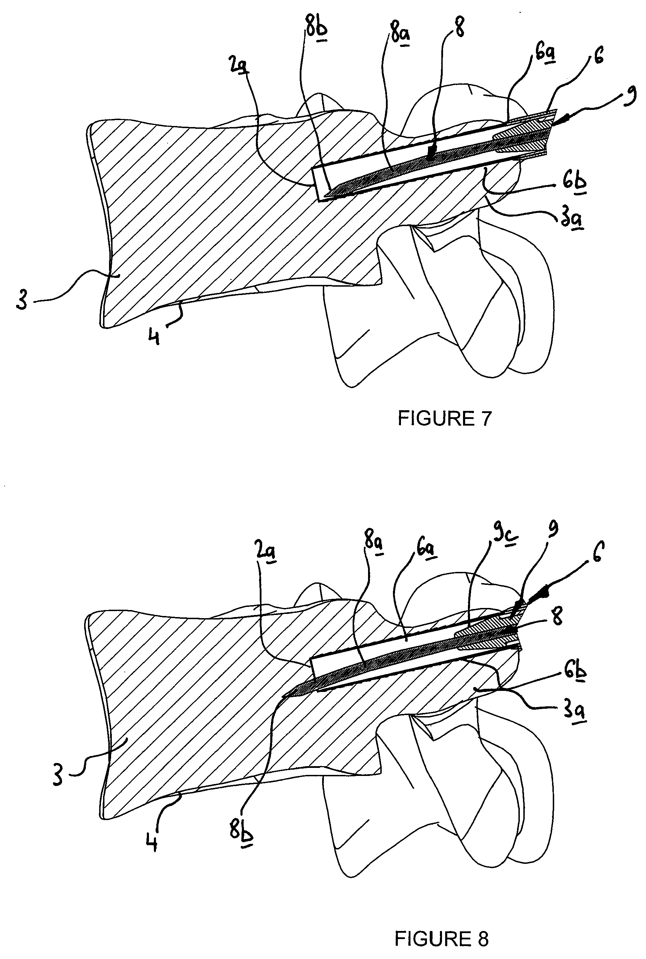

Le porte broche 9 muni de la broche de guidage 8 sont introduits à l'intérieur de la canule droite 6, jusqu' à ce que l'extrémité courbe 8a de ladite broche de guidage soit logée à l'intérieur du canal droit 2a. L'extrémité courbe 8a est contrainte à l'intérieur du tube 6a de la canule droite 6 du fait des caractéristiques d'élasticité de la broche de guidage 8 (Figure 7).

Le porte broche 9 est déplacé en translation à l'intérieur du tube 6a de la canule droite 6 jusqu'à ce que l'extrémité libre à profil conique 9c pénètre dans l'os spongieux du corps de la vertèbre 3 (Figures 8 à 11).

Le déplacement du porte broche 9 à l'intérieur du tube 6a de la canule droite 6 permet d'amener l'extrémité effilée 8b de la broche de guidage 8 à l'extérieur dudit tube 6a afin que cette dernière pénètre à l'intérieur de l'os spongieux de la vertèbre 3.

Le profil courbe 8a de la broche de guidage 8, du fait de ses caractéristiques élastiques, reprend progressivement son rayon de courbure R, au fur et à mesure de l'avancement du porte broche 9 dans le tube 6a. Ainsi, la pointe effilée 8b de la broche de guidage se dirige progressivement en direction du plateau cortical 4 de la vertèbre 3 pour venir juste au dessus du disque intervertébral 5 (Figures 8 à 10).

Le porte broche 9 est déplacé en translation de manière que son extrémité libre à profil conique 9c pénètre dans l'os spongieux de la vertèbre 3 permettant à la broche de guidage 8 et plus particulièrement à son profil courbe 8a de reprendre son rayon de courbure initial R et ce en traversant le plateau cortical 4 en direction du disque intervertébral 5 (Figure 11).

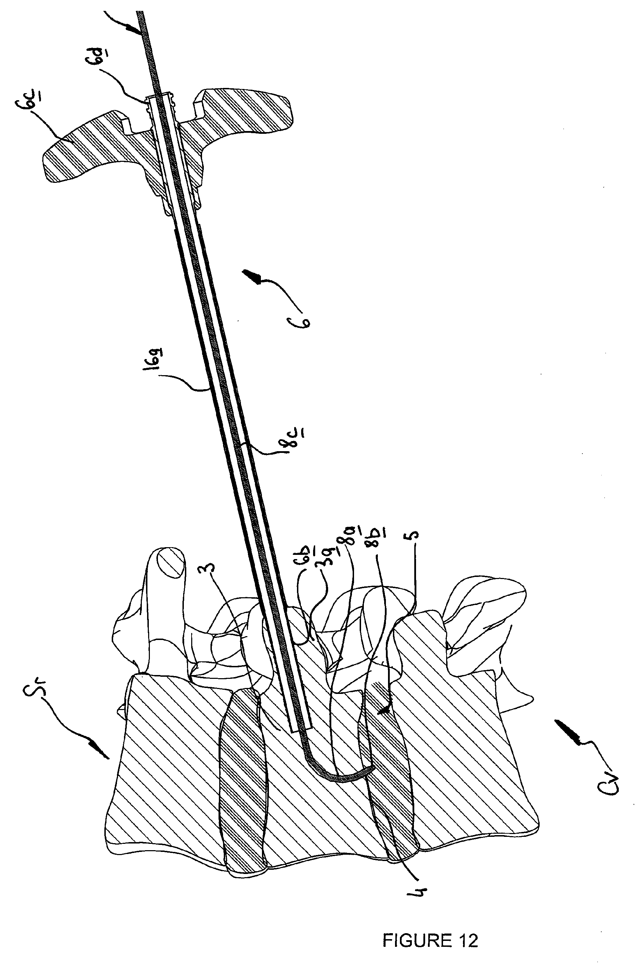

La molette de serrage 9e du porte broche 9 est déverrouillée pour permettre le retrait dudit porte broche de la canule droite 6 tout en laissant dans la vertèbre 3 et à l'intérieur de ladite canule droite 6 la broche de guidage 8 (Figure 12).

La canule droite 6 est ensuite légèrement dévissée du corps vertébral 3 pour laisser dans l'os spongieux une partie libre du canal osseux droit 2a dans le prolongement du tube 6a de ladite canule (Figure 13).

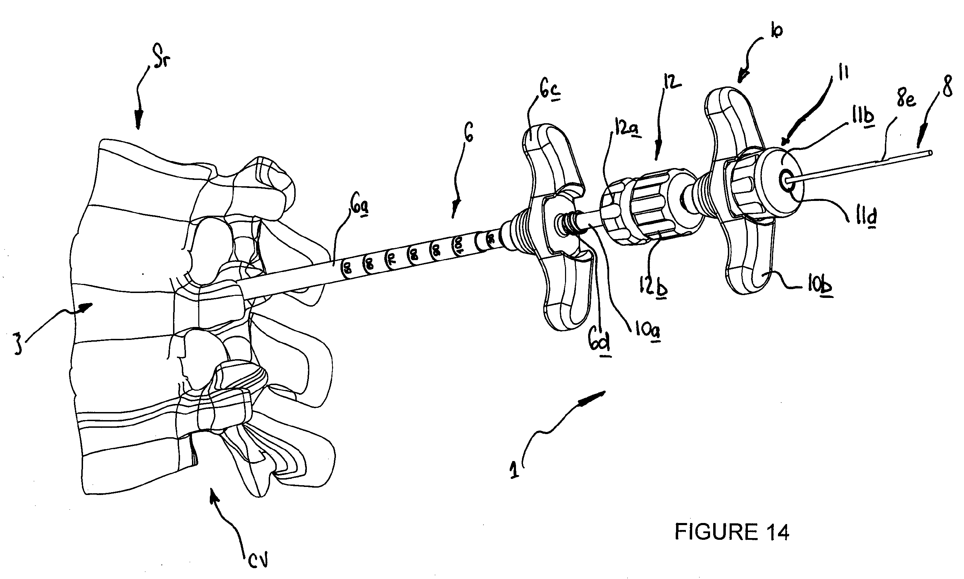

On a montré en Figures 14 à 20 les différentes étapes d'introduction du foret articulé 10 du dispositif de forage 1 permettant la réalisation d'un canal à profil courbe 2b dans le corps vertébral d'une vertèbre 3 pour atteindre la face supérieure du disque intervertébral 5.

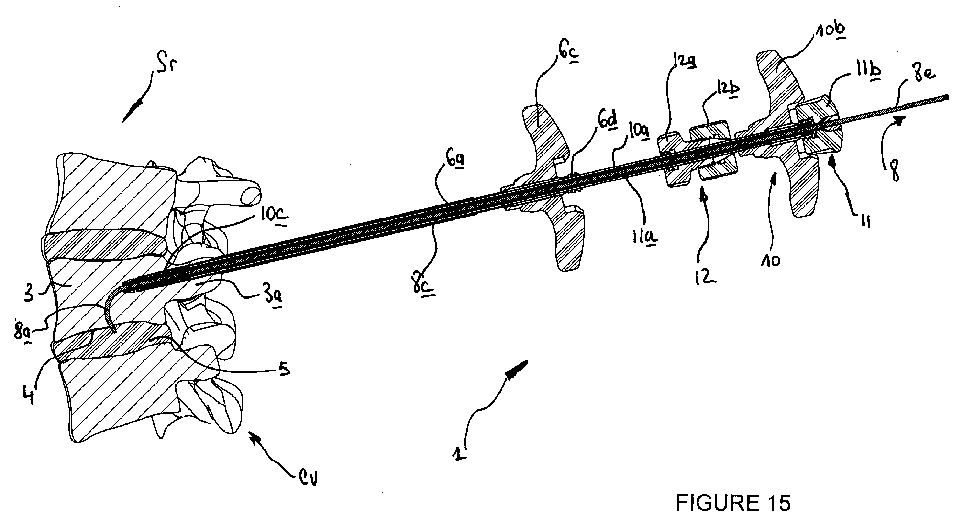

La gaine de centrage 11 est introduite à l'intérieur du tube métallique 10a du foret articulé 10 de manière que sa tête de préhension 11 b soit vissée sur le manchon cylindrique 10j de la poignée 10b dudit foret articulé (Figures 14 et 15).

Le dispositif de verrouillage 12 constitué de l'élément de retenue 12a et de l'écrou de serrage 12b est placé autour du tube métallique 10a du foret articulé 10 juste en dessous de la poignée de préhension 10b (Figures 14 et 15).

Le foret articulé 10 muni de sa gaine de centrage 11 et de son dispositif de verrouillage 12 est introduit dans le tube cylindrique 6a de la canule droite 6 de manière que la broche de guidage 8 soit placée à l'intérieur du tube en matière plastique 11a de ladite gaine disposée dans le tube métallique 10a dudit foret (Figures 14 et 15).

Le tube métallique 10a du foret articulé 10 est amené jusqu'au bout du canal droit 2a ménagé dans l'os spongieux du corps de la vertèbre 3, de manière que les séries de dents 10e et 10g soient en contact avec ledit os spongieux (Figure 15).

Ensuite, le tube métallique 10a est entrainé en rotation et en translation suivant une poussée au moyen de la poignée de préhension 10b à l'intérieur de la canule droite 6 de manière que les séries de dents 10e et 10g pénètrent à l'intérieur de l'os spongieux de la vertèbre 3. Le foret articulé 10 est guidé dans ses déplacements à l'intérieur de l'os spongieux par l'intermédiaire de la broche de guidage 8 afin de faciliter le forage du canal courbe 2b.

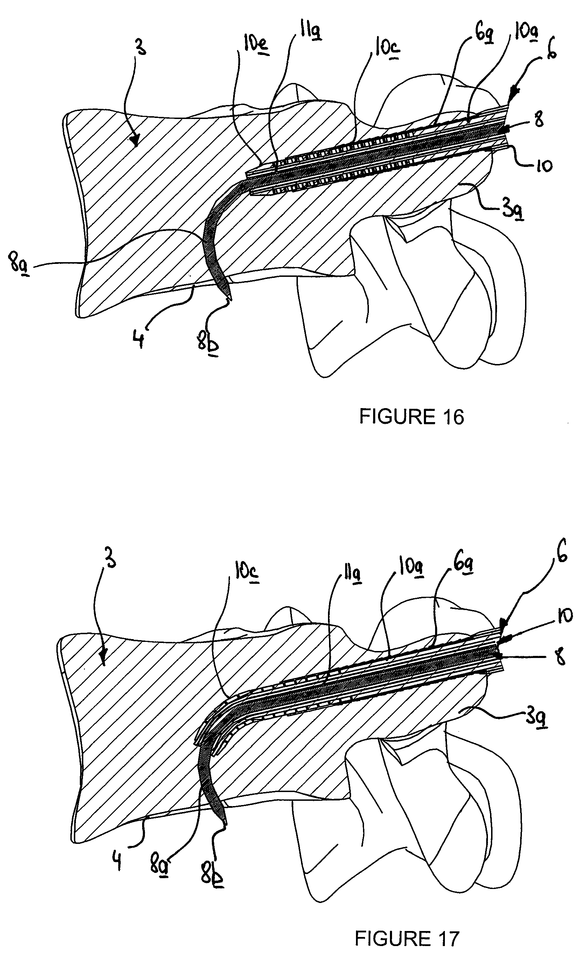

Le foret articulé 10 se déforme au niveau de son extrémité libre suivant le rayon de courbure R de la broche de guidage 8 du fait de l'agencement et des découpes ménagées dans le tube métallique 10a constituant le profil 10c (Figures 16 à 18).

Le foret articulé 10 permet le forage du canal courbe 2b à l'intérieur de l'os spongieux de la vertèbre 3 afin de déboucher suivant une direction sensiblement perpendiculaire au niveau du plateau inférieur formé par le plateau cortical 4 et donc au dessus du disque intervertébral 5.

Le redressement du foret articulé 10 afin que celui-ci débouche suivant une direction sensiblement perpendiculaire dans le plateau corticale 4 et donc à l'intérieur du disque intervertébral 5 est obtenu par les dimensions du rayon de courbure R et de l'angle Y de la broche de guidage 8.

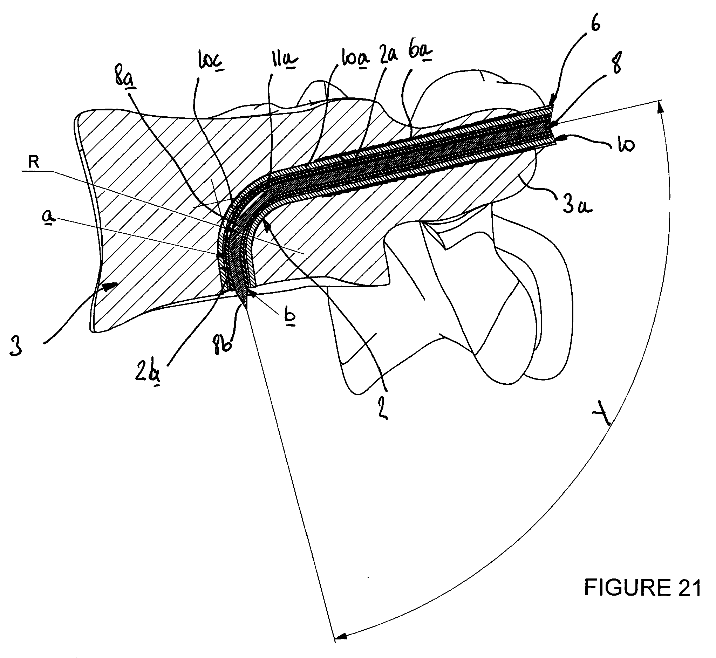

Pour cela, le rayon de courbure R de la broche de guidage 8 permet de définir, après son introduction dans le corps vertébral 3 et au niveau de son profil courbe 8a, deux points de contact a et b assurant le guidage de l'extrémité libre 10c du foret articulé 10 pour le positionner pendant le forage dans une direction sensiblement perpendiculaire au plateau cortical 4 de la vertèbre 3 à percer (Figure 21).

Le premier point de contact a est défini par la tangente au profil externe du rayon courbure R de la broche de guidage 8 qui est perpendiculaire au plateau cortical 4 de la vertèbre 3, tandis que le second point de contact b est défini par l'extrémité libre du foret articulé 10 qui vient contre le profil interne de la pointe effilée 8b de la broche de guidage 8 se trouvant dans l'épaisseur du plateau cortical 4 de la vertèbre 3 (Figure 21).

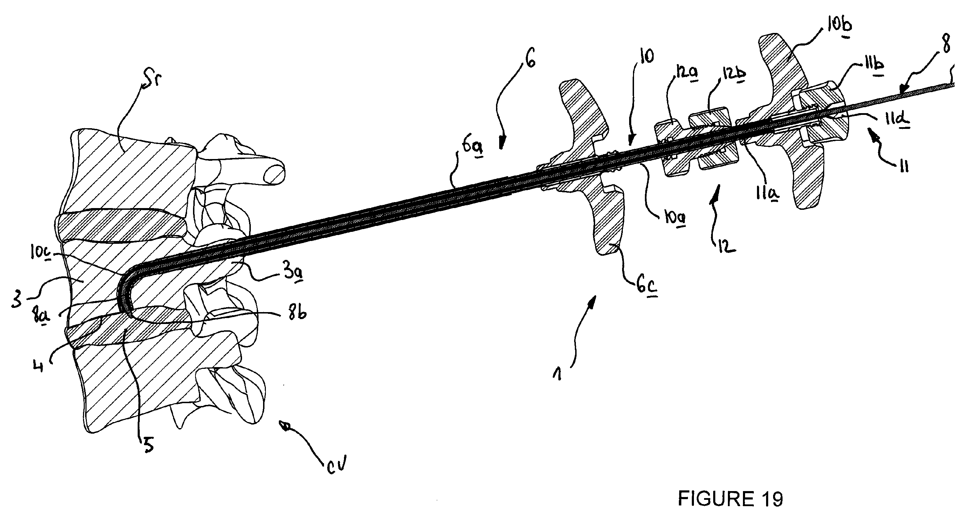

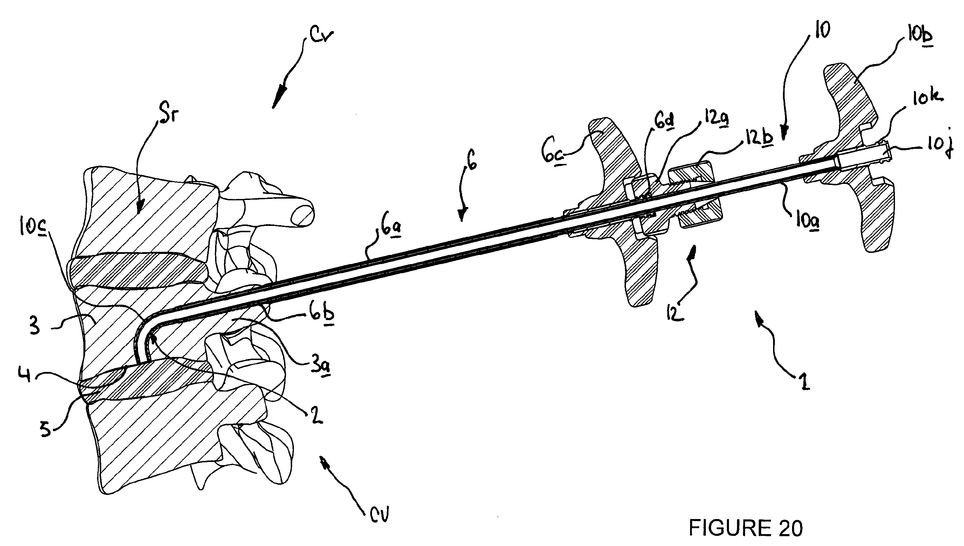

Lorsque le foret articulé 10 a percé le plateau cortical 4 de la vertèbre 3, ce dernier est maintenu en position et immobilisé sur la canule droite 6 au moyen du dispositif de verrouillage 12. Pour cela, l'élément de retenue 12a est vissé sur le profil fileté 6d de la canule droite 6, tandis que l'écrou de serrage 12b est serré sur l'élément de retenue 12a afin d'immobiliser le dispositif de verrouillage 12 par pincement sur le tube métallique 10a du foret articulé 10 (Figures 19 et 20).

Le verrouillage du foret articulé 10 sur la canule droite 6 permet d'éviter tout déplacement dudit foret lors de l'extraction de la broche de guidage 8 et de la gaine de centrage 11 (Figure 20).

Dès que le foret articulé 10 est libéré de la broche de guidage 8 et de la gaine de centrage 11, il permet l'accès au moyen de son canal interne à profil courbe à d'autres instruments non représentés assurant, par exemple, la nucléotomie du disque intervertébral 5.

Il doit d'ailleurs être entendu que la description qui précède n'a été donnée qu'à titre d'exemple, et qu'elle ne limite nullement le domaine de l'invention dont on ne sortirait pas en remplaçant les détails d'exécution décrits par tout autre équivalent.

## Revendications

1. Dispositif de forage pour la réalisation d'un canal osseux (2) à profil courbe (2b) au travers d'une canule droite (6) préalablement ancrée dans le corps d'une vertèbre (3) pourvue d'un plateau cortical (4) d'un segment rachidien (Sr) d'une colonne vertébrale (Cv), **caractérisé en ce qu'**il comporte une broche de guidage (8) issue d'un kit de broche de guidage et un foret articulé (10) constitué d'un tube cylindrique (10a) comportant une extrémité agencée suivant un profil (10c) assurant la déformation et l'articulation suivant une forme courbe, ladite broche de guidage (8) présentant à l'une de ses extrémités d'une part un profil courbe (8a) dont le rayon de courbure R est inférieur à 20 millimètres et d'autre part une pointe effilée (8b) se trouvant dans une direction définie par un angle Y qui est inférieur à 90 degrés par rapport à l'axe longitudinal de ladite broche, le profil de ladite broche de guidage (8) permettant de définir, après son introduction dans le corps de la vertèbre (3), un premier point de contact **a** entre la broche (8) et le foret articulé (10),_ledit point de contact **a** étant positionné au niveau de la tangente au profil externe du rayon de courbure R de la broche de guidage (8) dans une direction perpendiculaire au plateau cortical (4), et un second point de contact **b** entre la broche (8) et le foret articulé (10), ledit point de contact **b** étant positionnée à l'extrémité libre dudit foret qui vient contre le profil externe de la pointe effilée (8b) de la broche de guidage (8) se trouvant dans l'épaisseur du plateau cortical (4), lesdits deux points de contact **a** et **b** assurant le guidage de l'extrémité libre du foret articulé (10) afin de positionner ladite extrémité libre dans une direction sensiblement perpendiculaire à celle du plateau cortical (4) de la vertèbre (3) à percer.

2. Dispositif de forage suivant la revendication 1, **caractérisé en ce que** chaque broche de guidage (8) est réalisée dans un matériau à la fois résistant et flexible tel que dans un alliage en Nitinol super élastique.

3. Dispositif de forage suivant la revendication 1, **caractérisé en ce que** chaque broche de guidage (8) présente un diamètre externe qui est inférieur à 3 millimètres

4. Dispositif de forage suivant la revendication 3, **caractérisé en ce que** chaque broche de guidage (8) présente un diamètre externe qui est compris entre 1.4 millimètres et 2 millimètres.

5. Dispositif de forage suivant la revendication 1, **caractérisé en ce que** le profil courbe (8a) présente un rayon de courbure R qui est compris entre 10 millimètres et 20 millimètres.

6. Dispositif de forage suivant la revendication 1, **caractérisé en ce que** l'angle Y de la pointe effilé (8b) est compris entre 70 et 85 degrés.

7. Dispositif de forage suivant la revendication 1, **caractérisé en ce que** chaque broche de guidage (8) comporte une partie longitudinale droite (8c) présentant un méplat (8d) coopérant avec un profil complémentaire (9a) ménagé dans un porte broche (9) permettant d'assurer d'une part le blocage en rotation de la broche de guidage (8) à l'intérieur du porte broche (9) et d'autre part de rigidifier ladite broche de guidage (8) sur sa partie longitudinale droite (8c) lors de l'introduction de ladite broche de guidage (8) et dudit porte broche (9) au travers d'une canule droite (6) préalablement ancrée dans le corps de la vertèbre (3).

8. Dispositif de forage suivant la revendication 7, **caractérisé en ce que** chaque broche de guidage (8) comporte au niveau de son extrémité opposée à celle à profil courbe (8a) une encoche (8e) ménagée dans la partie longitudinale droite (8c) et plus particulièrement du coté du méplat (8d), ladite encoche (8e) coopérant avec une mollette de serrage (9e) guidée dans une poignée de préhension (9d) du porte broche (9) pour le blocage en translation de ladite broche de guidage (8) dans ledit porte broche (9).

9. Dispositif de forage suivant la revendication 7, **caractérisé en ce que** le porte broche (9) est constitué d'un tube cylindrique et métallique (9b) solidaire à l'une de ses extrémités d'une poignée de préhension (9d) pourvue d'une molette de serrage (9e) et d'un manchon cylindrique creux (9f) présentant sur son profil externe un filetage rapide (9g) permettant le vissage d'un bouchon (9h) permettant de bloquer en translation la broche de guidage (8) par rapport au porte broche (9).

10. Dispositif de forage suivant la revendication 1, **caractérisé en ce que** le foret articulé (10) est constitué d'un tube cylindrique et métallique (10a) comportant à l'une de ses extrémités une poignée de préhension (10b), tandis que l'autre extrémité est découpée d'une part suivant un profil (10c) assurant la déformation et l'articulation suivant une forme courbe de ladite extrémité et d'autre part pour déterminer une extrémité coupante (10m) comprenant une première série de dents (10e) disposée sur la périphérie dudit tube et une seconde série de dents (10g) disposée en bout du tube métallique (10a) et d'une gaine de protection (10i) placée dans la partie interne du tube métallique (10a) ladite gaine de protection (10i) étant réalisée en matériau souple permettant de lisser intérieurement les aspérités et les discontinuités de l'extrémité libre provenant du profil (10c).

11. Dispositif de forage suivant la revendication 10, **caractérisé en ce que** la poignée de préhension (10b) comporte dans le prolongement du tube métallique (10a) un manchon cylindrique creux (10j) présentant sur son profil externe un filetage rapide (10k) permettant l'introduction et la fixation d'une gaine de centrage (11) à l'intérieur dudit tube métallique (10a).

12. Dispositif de forage suivant l'une quelconque des revendications 10 et 11, **caractérisé en ce que** la gaine de protection (10i) est réalisée dans un matériau souple assurant un coefficient de glissement important pour le coulissement de la broche de guidage (8) et/ou de la gaine de centrage (11) lors du forage du canal osseux (2) à profil courbe (2b).

13. Dispositif de forage suivant la revendication 10, **caractérisé en ce que** le profil (10c) du tube cylindrique et métallique (14a) est constitué d'une succession de boucles (10d) alternativement concaves et convexes assurant la déformation et l'articulation suivant une forme courbe de l'extrémité du foret articulé (10).

14. Dispositif de forage suivant la revendication 10, **caractérisé en ce que** le bord tranchant (10f) de chaque dent de la première série de dents (10e) présente une faible inclinaison par rapport à l'axe longitudinal du foret articulé (10).

15. Dispositif de forage suivant la revendication 10, **caractérisé en ce que** le bord tranchant (10h) de chaque dent de la seconde série de dents (10g) présente une forte inclinaison de manière à couper l'axe longitudinal du foret articulé (10).

16. Dispositif de forage suivant la revendication 11, **caractérisé en ce que** la gaine de centrage (11) est constituée d'un tube cylindrique (11a) réalisé dans un matériau souple solidaire à l'une de ses extrémités d'une tête de préhension (11b) comportant d'une part un alésage interne fileté (11c) destiné à coopérer avec le manchon (10j) de la poignée (10b) du foret articulé (10) pour son immobilisation sur ce dernier et d'autre part à l'opposé de l'alésage interne fileté (11c) un dispositif de blocage (11d) permettant par pincement, de bloquer en translation le tube cylindrique (11a) à l'intérieur de ladite tête de préhension (11 b) de la gaine de centrage (11).

17. Dispositif de forage suivant la revendication 11, **caractérisé en ce que** le foret articulé (10) comprend un dispositif de verrouillage (12) disposé sur le tube métallique (10a) et en dessous de la poignée de préhension (10b) afin de pouvoir au moyen d'un élément de retenue (12a) et d'un écrou de serrage (12b) immobiliser ledit foret sur la canule droite (6) préalablement ancrée dans le corps de la vertèbre (3).

18. Dispositif de forage suivant la revendication 17, **caractérisé en ce que** le dispositif de verrouillage (12) est constitué d'un élément de retenue (12a) comportant une tête cylindrique (12c) solidaire d'un manchon (12d) présentant un profil externe cylindrique (12e) pourvu d'un filetage (12f) se prolongeant par un profil externe conique fendu (12g).

19. Dispositif de forage suivant la revendication 18, **caractérisé en ce que** l'élément de retenue (12a) est percé en son milieu d'un alésage débouchant (12h) comportant au niveau de la tête cylindrique (12c) un filetage interne (12i) coopérant avec un profil fileté (6d) de la canule droite (6) pour l'immobilisation de l'élément de retenue (12) sur ladite canule.

20. Dispositif de forage suivant les revendications 17 et 18, **caractérisé en ce que** le dispositif de verrouillage (12) est constitué d'un écrou de serrage (12b) comportant dans sa partie interne un premier alésage fileté (12j) coopérant avec celui (12f) ménagé sur la partie cylindrique (12e) du manchon (12d) de l'élément de retenue (12a) et un second alésage interne (12k) coaxial au premier et présentant un profil conique d'inclinaison complémentaire à celui externe (12g) dudit manchon (12d).

21. Dispositif de forage suivant la revendication 10, **caractérisé en ce que** le foret articulé (10) comprend des moyens de sécurité (13) assurant la récupération de l'extrémité coupante (10m) en cas de rupture dudit foret articulé.

22. Dispositif de forage suivant les revendications 10, 16 et 21, **caractérisé en ce que** les moyens de sécurité (13) sont constitués d'un fil souple ou câble souple (13a) placé à l'intérieur du tube métallique (10a) et entre la gaine de protection (10i) solidaire de la face interne dudit tube et la face externe de la gaine de centrage (11) de manière que chaque extrémité (13b, 13c) dudit fil souple (13a) coopère avec la tête de préhension (11b) de ladite gaine de centrage (11).

23. Dispositif de forage suivant les revendications 10, 16 et 21, **caractérisé en ce que** les moyens de sécurité (13) sont constitués d'un fil souple ou câble souple (13a) placé à l'intérieur du tube métallique (10a) et entre la face interne de ce dernier et la gaine de protection (10i) de manière que chaque extrémité (13b, 13c) dudit fil souple (13a) coopère avec la tête de préhension (11b) de ladite gaine de centrage (11).

24. Dispositif de forage suivant la revendication 22, **caractérisé en ce que** les extrémités (13b, 13c) du fil souple de sécurité (13a) comportent respectivement une butée (13d) placée dans un logement (11f) de même profil ménagé dans la tête de préhension (11b) de la gaine de centrage (11).

## Patentansprüche

1. Bohrvorrichtung zur Herstellung eines Knochenkanals (2) mit gewölbtem Profil (2b) durch eine zuvor in einem Wirbelkörper (3) verankerte gerade Kanüle (6), bereitgestellt mit einer Kortikalisplatte (4) eines Wirbelsegments (Sr) einer Wirbelsäule (Cv), **dadurch gekennzeichnet, dass** sie einen Führungsstift (8) aus einem Satz an Führungsstiften und einen artikulierten Bohrer (10) umfasst, bestehend aus einem zylindrischen Rohr (10a) mit einem Ende, das einem Profil (10c) folgend angeordnet ist, das die Verformung und die Artikulation gemäß einer gewölbten Form sicherstellt, wobei der Führungsstift (8) an einem seiner Enden einerseits ein gewölbtes Profil (8a), dessen Krümmungsradius R geringer als 20 Millimeter ist, und andererseits eine abgeschrägte Spitze (8b) aufweist, die sich in einer Richtung befindet, die durch einen Winkel Y definiert ist, der in Bezug auf die Längsachse des Stiftes geringer als 90 Grad ist, wobei das Profil des Führungsstiftes (8) nach seiner Einführung in den Wirbelkörper (3) ermöglicht, einen ersten Kontaktpunkt **a** zwischen dem Stift (8) und dem artikulierten Bohrer (10), wobei sich der Kontaktpunkt **a** auf Höhe der Tangente zum Außenprofil des Krümmungsradius R des Führungsstiftes (8) in einer Richtung senkrecht zur Kortikalisplatte (4) befindet, und einen zweiten Kontaktpunkt **b** zwischen dem Stift (8) und dem artikulierten Bohrer (10) zu definieren, wobei sich der Kontaktpunkt **b** an dem freien Ende des Bohrers befindet, das an das Außenprofil der abgeschrägten Spitze (8b) des Führungsstiftes (8) anliegt, der sich in der Dicke der Kortikalisplatte (4) befindet, wobei die zwei Kontaktpunkte **a** und **b** die Führung des freien Endes des artikulierten Bohrers (10) zum Positionieren des freien Endes in einer Richtung, die im Wesentlichen senkrecht zu derjenigen der Kortikalisplatte (4) des zu durchbohrenden Wirbels (3) ist, sicherstellen.

2. Bohrvorrichtung nach Anspruch 1, **dadurch gekennzeichnet, dass** jeder Führungsstift (8) aus einem Material besteht, das gleichzeitig widerstandsfähig und flexibel wie bei einer superelastischen Nitinol-Legierung ist.

3. Bohrvorrichtung nach Anspruch 1, **dadurch gekennzeichnet, dass** jeder Führungsstift (8) einen Außendurchmesser aufweist, der geringer als 3 Millimeter ist.

4. Bohrvorrichtung nach Anspruch 3, **dadurch gekennzeichnet, dass** jeder Führungsstift (8) einen Außendurchmesser aufweist, der zwischen 1,4 Millimeter und 2 Millimeter beträgt.

5. Bohrvorrichtung nach Anspruch 1, **dadurch gekennzeichnet, dass** das gewölbte Profil (8a) einen Krümmungsradius R aufweist, der zwischen 10 Millimeter und 20 Millimeter beträgt.

6. Bohrvorrichtung nach Anspruch 1, **dadurch gekennzeichnet, dass** der Winkel Y der abgeschrägten Spitze (8b) zwischen 70 und 85 Grad beträgt.

7. Bohrvorrichtung nach Anspruch 1, **dadurch gekennzeichnet, dass** jeder Führungsstift (8) einen geraden Längsabschnitt (8c) mit einer Abflachung (8d) aufweist, mit einem komplementären Profil (9a) kooperierend, das in einer Spindel (9) vorgesehen ist, um einerseits die Blockierung der Drehung des Führungsstiftes (8) im Inneren der Spindel (9) sicherstellen zu können und andererseits den Führungsstift (8) bei der Einführung des Führungsstiftes (8) und der Spindel (9) durch eine rechte Kanüle (6), die zuvor in einem Wirbelkörper (3) verankert wurde, auf dem geraden Längsabschnitt (8c) zu versteifen.

8. Bohrvorrichtung nach Anspruch 7, **dadurch gekennzeichnet, dass** jeder Führungsstift (8) an seinem Ende gegenüber demjenigen mit gewölbtem Profil (8a) eine Kerbe (8e) umfasst, die im geraden Längsabschnitt (8c) und insbesondere auf der Seite der Abflachung (8d) vorgesehen ist, wobei die Kerbe (8e) mit einem Spannrad (9e) kooperiert, das in einen Handgriff (9d) der Spindel (9) zur Blockierung der Translation des Führungsstiftes (8) in der Spindel (9) geführt ist.

9. Bohrvorrichtung nach Anspruch 7, **dadurch gekennzeichnet, dass** die Spindel (9) aus einem zylindrischen Metallrohr (9b) besteht, das an einem seiner Enden mit einem Handgriff (9d) mit einem Spannrad (9e) und einer zylindrischen Hohlmuffe (9f) verbunden ist, aufweisend auf ihrem Außenprofil ein Schnellgewinde (9g), um die Verschraubung eines Verschlusses (9h) zur Ermöglichung der Blockierung der Translation des Führungsstiftes (8) im Verhältnis zur Spindel (9) zu ermöglichen.

10. Bohrvorrichtung nach Anspruch 1, **dadurch gekennzeichnet, dass** der artikulierte Bohrer (10) ein zylindrisches Metallrohr (10a) umfasst, das an einem Ende einen Handgriff (10b) aufweist, während das andere Ende einerseits gemäß einem Profil (10c) geschnitten ist, das die Verformung und Artikulation des Endes gemäß einer gewölbten Form sicherstellt, und andererseits zum Bestimmen eines schneidenden Endes (10m), das eine erste Reihe an Zähnen (10e) angeordnet am Rand des Rohres und eine zweite Reihe an Zähnen (10g) angeordnet am Ende des Metallrohres (10a) und ein Schutzrohr (10i) platziert im Innenteil des Metallrohres (10a) umfasst, wobei das Schutzrohr (10i) aus einem flexiblen Material besteht, das ermöglicht, Unebenheiten und Unterbrechungen des freien Endes aus dem Profil (10c) intern zu glätten.

11. Bohrvorrichtung nach Anspruch 10, **dadurch gekennzeichnet, dass** der Handgriff (10b) in der Verlängerung des Metallrohres (10a) eine zylindrische Hohlmuffe (10j) aufweist, die an ihrem Außenprofil ein Schnellgewinde (10k) aufweist, das die Einführung und die Befestigung einer Zentrierhülle (11) im Inneren des Metallrohres (10a) ermöglicht.

12. Bohrvorrichtung nach einem der Ansprüche 10 und 11, **dadurch gekennzeichnet, dass** das Schutzrohr (10i) aus einem flexiblen Material besteht, das einen großen Reibungskoeffizienten zum Verschieben des Führungsstiftes (8) und/oder der Zentrierhülle (11) beim Bohren des Knochenkanals (2) mit gewölbtem Profil (2b) sicherstellt.

13. Bohrvorrichtung nach Anspruch 10, **dadurch gekennzeichnet, dass** das Profil (10c) des zylindrischen Metallrohres (10a) aus einer Folge von Schlaufen (10d) besteht, die abwechselnd konkav und konvex sind und eine Verformung und Artikulation gemäß einer gewölbten Form des Endes des artikulierten Bohrers (10) sicherstellen.

14. Bohrvorrichtung nach Anspruch 10, **dadurch gekennzeichnet, dass** der Schneiderand (10f) jedes Zahns aus der ersten Reihe an Zähnen (10e) eine schwache Neigung in Bezug auf die Längsachse des artikulierten Bohrers (10) aufweist.

15. Bohrvorrichtung nach Anspruch 10, **dadurch gekennzeichnet, dass** der Schneiderand (10h) jedes Zahns aus der zweiten Reihe an Zähnen (10g) eine starke Neigung zum Schneiden der Längsachse des artikulierten Bohrers (10) aufweist.

16. Bohrvorrichtung nach Anspruch 11, **dadurch gekennzeichnet, dass** die Zentrierhülle (11) aus einem zylindrischen Rohr (11a) besteht, das aus einem flexiblen Material besteht, das an einem der Enden mit einem Greiferkopf (11 b) verbunden ist, umfassend einerseits eine Schraubinnenbohrung (11 c) zum Kooperieren mit der Muffe (10j) des Griffs (10b) des artikulierten Bohrers (10) zur Immobilisierung auf Letzterem und andererseits gegenüber der Schraubinnenbohrung (11c) eine Blockiervorrichtung (11d) zum Ermöglichen der Blockierung der Translation des zylindrischen Rohrs (11a) im Inneren des Greiferkopfes (11 b) der Zentrierhülle (11) durch Einklemmen.

17. Bohrvorrichtung nach Anspruch 11, **dadurch gekennzeichnet, dass** der artikulierte Bohrer (10) eine Verriegelungsvorrichtung (12) umfasst, die auf dem Metallrohr (10a) und unter dem Haltegriff (10b) angeordnet ist, um mittels eines Rückhaltemittels (12a) und einer Klemmmutter (12b) den Bohrer auf der rechten Kanüle (6), die zuvor in einem Wirbelkörper (3) verankert wurde, immobilisieren zu können.

18. Bohrvorrichtung nach Anspruch 17, **dadurch gekennzeichnet, dass** die Verriegelungsvorrichtung (12) ein Rückhalteelement (12a) mit einem zylindrischen Kopf (12c) umfasst, der mit einer Muffe (12d) verbunden ist, die ein zylindrisches Außenprofil (12e) mit einem Gewinde (12f), das sich durch ein konisch gespaltenes Außenprofil (12g) erstreckt, aufweist.

19. Bohrvorrichtung nach Anspruch 18, **dadurch gekennzeichnet, dass** das Rückhalteelement (12a) in seiner Mitte mit einer Durchgangsbohrung (12h) durchbohrt ist, auf der Ebene des zylindrischen Kopfes (12c) ein Innengewinde (12i) umfassend, das mit einem Schraubprofil (6d) der geraden Kanüle (6) zur Immobilisierung des Rückhalteelements (12) auf der Kanüle kooperiert.

20. Bohrvorrichtung nach den Ansprüchen 17 und 18, **dadurch gekennzeichnet, dass** die Verriegelungsvorrichtung (12) aus einer Klemmmutter (12b) besteht, die im inneren Teil eine erste Gewindebohrung (12j) in Kooperation mit derjenigen (12f), die auf dem zylindrischen Teil (12e) der Muffe (12d) des Rückhalteelements (12a) vorgesehen ist, und eine zweite interne Bohrung (12k) koaxial zur ersten und mit einem konischen Neigungsprofil komplementär zu demjenigen äußeren (12g) der Muffe (12d) aufweist.

21. Bohrvorrichtung nach Anspruch 10, **dadurch gekennzeichnet, dass** der artikulierte Bohrer (10) Sicherungsmittel (13) umfasst, die die Rückgewinnung des schneidenden Endes (10m) im Falle eines Bruchs des artikulierten Bohrers sicherstellen.

22. Bohrvorrichtung nach den Ansprüchen 10, 16 und 21, **dadurch gekennzeichnet, dass** die Sicherungsmittel (13) aus einem flexiblen Draht oder flexiblen Kabel (13a) bestehen, der oder das im Inneren des Metallrohres (10a) und zwischen dem Schutzrohr (10i) verbunden mit der Innenfläche des Rohres und der Außenfläche der Zentrierhülle (11) auf solche Weise platziert ist, dass jedes Ende (13b, 13c) des flexiblen Drahtes (13a) mit dem Greiferkopf (11 b) der Zentrierhülle (11) kooperiert.

23. Bohrvorrichtung nach den Ansprüchen 10, 16 und 21, **dadurch gekennzeichnet, dass** die Sicherungsmittel (13) aus einem flexiblen Draht oder flexiblen Kabel (13a) bestehen, der oder das im Inneren des Metallrohres (10a) und zwischen der Innenfläche von Letzterem und der Schutzhülle (10i) auf solche Weise platziert ist, dass jedes Ende (13b, 13c) des flexiblen Drahtes (13a) mit dem Greiferkopf (11 b) der Zentrierhülle (11) kooperiert.

24. Bohrvorrichtung nach Anspruch 22, **dadurch gekennzeichnet, dass** die Enden (13b, 13c) des flexiblen Sicherungsdrahtes (13a) jeweils einen Anschlag (13d) angeordnet in einem Gehäuse (11f) des gleichen Profils, das im Greiferkopf (11b) der Zentrierhülle (11) vorgesehen ist, umfassen.

## Claims

1. Drilling device for making a bone canal (2) with a curved profile (2b) through a straight cannula (6) anchored beforehand in the body of a vertebra (3) provided with a cortical end-plate (4) of a spinal segment (Sr) of a vertebral column (Cv), **characterized in that** it comprises a guide pin (8) from a guide pin kit and an articulated drill bit (10) consisting of a cylindrical tube (10a) comprising an end arranged in accordance with a profile (10c) ensuring the deformation and the articulation in accordance with a curved shape, said guide pin (8) having at one of its ends, on the one hand, a curved profile (8a) whose radius of curvature R is less than 20 millimeters, and, on the other hand, a tapered tip (8b) extending in a direction defined by an angle Y that is less than 90 degrees relative to the longitudinal axis of said pin, the profile of said guide pin (8) making it possible to define, after its introduction into the body of the vertebra (3), a first contact point **a** between the pin (8) and the articulated drill bit (10), said contact point **a** being positioned on the tangent to the external profile of the radius of curvature R of the guide pin (8) in a direction perpendicular to the cortical end-plate (4), and a second contact point **b** between the pin (8) and the articulated drill bit (10), said contact point **b** being positioned at the free end of said drill bit which abuts against the external profile of the tapered tip (8b) of the guide pin (8) extending in the thickness of the cortical end-plate (4), said two contact points **a** and b ensuring the guiding of the free end of the articulated drill bit (10) in order to position said free end in a direction that is substantially perpendicular to that of the cortical end-plate (4) of the vertebra (3) to be perforated.

2. Drilling device according to Claim 1, **characterized in that** each guide pin (8) is made of a material that is both resistant and flexible, such as a super-resilient Nitinol alloy.

3. Drilling device according to Claim 1, **characterized in that** each guide pin (8) has an external diameter that is less than 3 millimeters.

4. Drilling device according to Claim 3, **characterized in that** the guide pin (8) has an external diameter which is between 1.4 millimeters and 2 millimeters.

5. Drilling device according to Claim 1, **characterized in that** the curved profile (8a) has a radius of curvature R between 10 millimeters and 20 millimeters.

6. Drilling device according to Claim 1, **characterized in that** the angle Y of the tapered tip (8b) is between 70 and 85 degrees.

7. Drilling device according to Claim 1, **characterized in that** each guide pin (8) comprises a straight longitudinal portion (8c) having a flat spot (8d) cooperating with a complementary profile (9a) made in a pin support (9), making it possible to ensure, on the one hand, the rotational blocking of the guide pin (8) inside the pin support (9), and, on the other hand, the stiffening of said guide pin (8) in its straight longitudinal portion (8c) when said guide pin (8) and said pin support (9) are introduced through a straight cannula (6) anchored beforehand in the body of the vertebra (3).

8. Drilling device according to Claim 7, **characterized in that** each guide pin (8) comprises at the site of its end opposite the end with a curved profile (8a), a notch (8e) made in the straight longitudinal portion (8c) and, more particularly, on the side of the flat area (8d), said notch (8e) cooperating with a tightening wheel (9e) guided in a gripping handle (9d) of the pin support (9) for the rotational blocking of said guide pin (8) in said pin support (9).

9. Drilling device according to Claim 7, **characterized in that** the pin support (9) consists of a cylindrical metal tube (9b) firmly connected at one of its ends to a gripping handle (9d) provided with a tightening wheel (9e) and a hollow cylindrical sleeve (9f) having, on its external profile, a quick threading (9g) making it possible to screw on a plug (9h) making it possible to achieve rotational blocking of the guide pin (8) relative to the pin support (9).

10. Drilling device according to Claim 1, **characterized in that** the articulated drill bit (10) consists of a cylindrical metal tube (10a) comprising at one of its ends a gripping handle (10b), while the other end is cut, on the one hand, in accordance with a profile (10c) ensuring the deformation and the articulation in accordance with a curved shape of said end, and, on the other hand, in order to determine a cutting end (10m) including a first series of teeth (10e) arranged on the periphery of said tube and a second series of teeth (10g) arranged at the end of the metal tube (10a), and of a protective sheath (10i) placed in the internal portion of the metal tube (10a), said protective sheath (10i) being made of a flexible material making it possible to smooth irregularities inside and discontinuities of the free end originating from the profile (10c).

11. Drilling device according to Claim 10, **characterized in that** the gripping handle (10b) comprises, in the extension of the metal tube (10a), a hollow cylindrical sleeve (10j) having on its external profile a quick threading (10k) making it possible to introduce and fasten a centering sheath (11) inside said metal tube (10a).

12. Drilling device according to either of Claims 10 and 11, **characterized in that** the protective sheath (10i) is made from a flexible material ensuring a high sliding coefficient for the sliding of the guide pin (8) and/or of the centering sheath (11) during the drilling of the bone canal (2) with a curved profile (2b).

13. Drilling device according to Claim 10, **characterized in that** the profile (10c) of the cylindrical metal tube (10a) consists of a succession of alternatingly concave and convex loops (10d), ensuring the deformation and the articulation in accordance with a curved shape of the end of the articulated drill bit (10).

14. Drilling device according to Claim 10, **characterized in that** the cutting edge (10f) of each tooth of the first series of teeth (10e) has a small inclination relative to the longitudinal axis of the articulated drill bit (10).

15. Drilling device according to Claim 10, **characterized in that** the cutting edge (10h) of each tooth of the second series of teeth (10g) has a large inclination so as to cut the longitudinal axis of the articulated drill bit (10).

16. Drilling device according to Claim 11, **characterized in that** the centering sheath (11) consists of a cylindrical tube (11a) made from a resilient material firmly connected at one of its ends to a gripping head (11b) comprising, on the one hand, a threaded internal borehole (11c) used to cooperate with the sleeve (10j) of the handle (10b) of the articulated drill bit (10) for its immobilization on the latter, and, on the other hand, opposite from the tapered internal borehole (11c), a blocking device (11d) making it possible, by pinching, to achieve a translational blocking of the cylindrical tube (11a) inside said gripping head (11b) of the centering sheath (11).

17. Drilling device according to Claim 11, **characterized in that** the articulated drill bit (10) includes a locking device (12) arranged on the metal tube (10a) and below the gripping handle (10b) so that it is possible, by means of a retaining element (12a) and a tightening nut (12b), to immobilize said drill bit on the right cannula (6) anchored beforehand in the body of the vertebra (3).

18. Drilling device according to Claim 17, **characterized in that** the locking device (12) consists of a retaining element (12a) comprising a cylindrical head (12c) firmly connected to a sleeve (12d) having a cylindrical external profile (12e) provided with a threading (12f) that is extended by a split conical external profile (12g).

19. Drilling device according to Claim 18, **characterized in that** the retaining element (12a) is perforated at its center with an open borehole (12h) comprising on the cylindrical head (12c) an internal threading (12i) cooperating with a threaded profile (6d) of the straight cannula (6) for the immobilization of the retaining element (12) on said cannula.

20. Drilling device according to Claims 17 and 18, **characterized in that** the locking device (12) consists of a tightening nut (12b) comprising in its internal portion a first threaded borehole (12j) cooperating with that (12f) made on the cylindrical portion (12e) of the sleeve (12d) of the retaining element (12a) and a second internal borehole (12k) coaxial to the first threaded borehole and having a conical profile with an inclination complementary to the external profile (12g) of said sleeve (12d).

21. Drilling device according to Claim 10, **characterized in that** the articulated drill bit (10) includes safety means (13) ensuring the recovery of the cutting end (10m) in the case in which said articulated drill bit breaks.

22. Drilling device according to Claims 10, 16 and 21, **characterized in that** the safety means (13) consist of a flexible wire or flexible cable (13a) placed inside the metal tube (10a) and between the protective sheath (10i) firmly connected to the internal surface of said tube and the external surface of said centering sheath (11) so that each end (13b, 13c) of said flexible wire (13a) cooperates with the gripping head (11 b) of said centering sheath (11).

23. Drilling device according to Claims 10, 16 and 21, **characterized in that** the safety means (13) consist of a flexible wire or flexible cable (13a) placed inside the metal tube (10a) and between the internal surface of the latter and the protective sheath (10i) so that each end (13b, 13c) of said flexible wire (13a) cooperates with the gripping head (11b) of said centering sheath (11).

24. Drilling device according to Claim 22, **characterized in that** the ends (13b, 13c) of the flexible safety wire (13a) comprise respectively a stop (13d) placed in a recess (11f) having the same profile made in the gripping head (11b) of the centering sheath (11).
